# EUROPEAN PATENT APPLICATION

(11) **EP 3 366 774 A1**
(43) Date of publication of application: **29.08.2018**
(21) Application number: 16857109.9
(22) Date of filing: 21.10.2016
(51) Int. Cl.: C12N 15/113, A61K 31/713, A61P 35/00, A61P 43/00, C12N 15/09

(54) **NUCLEIC ACID COMPLEX HAVING AT LEAST ONE BULGE STRUCTURE**

(30) Priority: 23.10.2015 JP 2015209207
(71) Applicant: Rena Therapeutics Inc., Tokyo 100-0004 (JP)
(72) Inventor: YANO, Junichi, Tokyo 1000004 (JP); TANIGAWARA, Kazuaki, Tokyo 1000004 (JP); YOKOTA, Takanori, Tokyo 113-8510 (JP); YOSHIOKA, Kotaro, Tokyo 113-8510 (JP)
(74) Representative: Aulich, Martin
(86) International application number: PCT/JP2016/004657
(87) International publication number: WO 2017/068791

(57) **Abstract**

[Problem to be Solved]

Provided is a nucleic acid complex, preferably a double-stranded nucleic acid complex, having an excellent effect of suppressing the expression of a target gene.

[Solution]

The problem is solved by using a nucleic acid complex, preferably a double-stranded nucleic acid complex, comprising an active moiety comprising an antisense nucleic acid complementary to a transcript, for example, a transcript of a target gene, and a carrier moiety comprising a nucleic acid complementary to the nucleic acid, and having at least one bulge structure.

SEQUENCE LISTING

<110>  Rena Therapeutics inc.

<120>  Nucleic acid complex comprising at least one bulge structure

<130>  F9365PCT

<160>  22

<170>  PatentIn version 3.5

<210>  1
<211>  16
<212>  DNA
<213>  Artificial Sequence

<220>
<223>  Artificially synthesized oligonucleotide sequence


<220>
<221>  modified_base
<222>  (1)..(2)
<223>  LNA

<220>
<221>  modified_base
<222>  (1)..(16)
<223>  Phosphorothioate bonds between nucleic acids

<220>
<221>  modified_base
<222>  (15)..(16)
<223>  LNA

<400>  1
ctcccagcgt gcgcca                                                       16


<210>  2
<211>  17
<212>  DNA
<213>  Artificial Sequence

<220>
<223>  Combined DNA/RNA: synthetic oligonucleotide


<220>
<221>  misc_feature
<222>  (9)..(9)
<223>  "u" at this position is RNA

<400>  2
tggcgcacug ctgggag                                                      17


<210>  3
<211>  18
<212>  DNA
<213>  Artificial Sequence

<220>
<223>  Combined DNA/RNA: synthetic oligonucleotide


<220>
<221>  misc_feature
<222>  (9)..(10)
<223>  "u" at these positions is RNA

<400>  3
tggcgcacuu gctgggag                                                    18


<210>  4
<211>  19
<212>  DNA
<213>  Artificial Sequence

<220>
<223>  Combined DNA/RNA: synthetic oligonucleotide


<220>
<221>  misc_feature
<222>  (9)..(11)
<223>  "u" at these positions is RNA

<400>  4
tggcgcacuu ugctgggag                                                   19


<210>  5
<211>  20
<212>  DNA
<213>  Artificial Sequence

<220>
<223>  Combined DNA/RNA: synthetic oligonucleotide


<220>
<221>  misc_feature
<222>  (9)..(12)
<223>  "u" at these positions is RNA

<400>  5
tggcgcacuu uugctgggag                                                  20


<210>  6
<211>  16
<212>  DNA
<213>  Artificial Sequence

<220>
<223>  Artificially synthesized oligonucleotide sequence


<220>
<221>  modified_base
<222>  (1)..(2)
<223>  LNA

<220>
<221>  modified_base
<222>  (1)..(16)
<223>  Phosphorothioate bonds between nucleic acids

<220>
<221>  modified_base
<222>  (15)..(16)
<223>  LNA

<400>  6
ctcccaacgt gcgcca                                                       16


<210>  7
<211>  16
<212>  DNA
<213>  Artificial Sequence

<220>
<223>  Artificially synthesized oligonucleotide sequence


<220>
<221>  modified_base
<222>  (1)..(2)
<223>  LNA

<220>
<221>  modified_base
<222>  (1)..(16)
<223>  Phosphorothioate bonds between nucleic acids

<220>
<221>  misc_feature
<222>  (7)..(7)
<223>  deoxy inosine

<220>
<221>  modified_base
<222>  (15)..(16)
<223>  LNA

<400>  7
ctcccancgt gcgcca                                                       16


<210>  8
<211>  16
<212>  DNA
<213>  Artificial Sequence

<220>
<223>  Artificially synthesized oligonucleotide sequence


<220>
<221>  modified_base
<222>  (1)..(16)
<223>  Phosphorothioate bonds between nucleic acids

<400>  8
ctcccaacgt gcgcca                                                       16

<210>  9
<211>  16
<212>  DNA
<213>  Artificial Sequence

<220>
<223>  Artificially synthesized oligonucleotide sequence


<220>
<221>  modified_base
<222>  (1)..(1)
<223>  2'-F RNA

<220>
<221>  modified_base
<222>  (1)..(16)
<223>  Phosphorothioate bonds between nucleic acids

<220>
<221>  modified_base
<222>  (16)..(16)
<223>  2'-F RNA

<400>  9
ctcccaacgt gcgcca                                                    16


<210>  10
<211>  16
<212>  DNA
<213>  Artificial Sequence

<220>
<223>  Artificially synthesized oligonucleotide sequence


<220>
<221>  modified_base
<222>  (1)..(2)
<223>  2'-F RNA

<220>
<221>  modified_base
<222>  (1)..(16)
<223>  Phosphorothioate bonds between nucleic acids

<220>
<221>  modified_base
<222>  (15)..(16)
<223>  2'-F RNA

<400>  10
cucccaacgt gcgcca                                                    16


<210>  11
<211>  16
<212>  DNA
<213>  Artificial Sequence

<220>
<223>  Artificially synthesized oligonucleotide sequence


<220>
<221>  modified_base
<222>  (1)..(3)
<223>  2'-F RNA

<220>
<221>  modified_base
<222>  (1)..(16)
<223>  Phosphorothioate bonds between nucleic acids

<220>
<221>  modified_base
<222>  (14)..(16)
<223>  2'-F RNA

<400>  11
cucccaacgt gcgcca                                                       16


<210>  12
<211>  16
<212>  DNA
<213>  Artificial Sequence

<220>
<223>  Artificially synthesized oligonucleotide sequence


<220>
<221>  modified_base
<222>  (1)..(4)
<223>  2'-F RNA

<220>
<221>  modified_base
<222>  (1)..(16)
<223>  Phosphorothioate bonds between nucleic acids

<220>
<221>  modified_base
<222>  (13)..(16)
<223>  2'-F RNA

<400>  12
cucccaacgt gcgcca                                                       16


<210>  13
<211>  21
<212>  DNA
<213>  Artificial Sequence

<220>
<223>  Artificially synthesized oligonucleotide sequence


<220>
<221>  misc_feature
<222>  (9)..(13)

<223>  "U" at these positions is RNA

<400>  13
tggcgcacuu uuugctggga g                                                21


<210>  14
<211>  22
<212>  DNA
<213>  Artificial Sequence

<220>
<223>  Artificially synthesized oligonucleotide sequence


<220>
<221>  misc_feature
<222>  (9)..(14)
<223>  "U" at these positions is RNA

<400>  14
tggcgcacuu uuuugctggg ag                                               22


<210>  15
<211>  20
<212>  DNA
<213>  Artificial Sequence

<220>
<223>  Artificially synthesized oligonucleotide sequence


<220>
<221>  misc_feature
<222>  (9)..(12)
<223>  deoxy inosine

<400>  15
tggcgcacnn nngctgggag                                                  20


<210>  16
<211>  20
<212>  DNA
<213>  Artificial Sequence

<220>
<223>  Artificially synthesized oligonucleotide sequence


<220>
<221>  misc_feature
<222>  (9)..(12)
<223>  inosine (RNA)

<400>  16
tggcgcacnn nngctgggag                                                  20


<210>  17
<211>  20
<212>  DNA

<213>  Artificial Sequence

<220>
<223>  Artificially synthesized oligonucleotide sequence

<400>  17
tggcgcactt ttgctgggag                                                    20


<210>  18
<211>  19
<212>  DNA
<213>  Artificial Sequence

<220>
<223>  Artificially synthesized oligonucleotide sequence


<220>
<221>  misc_feature
<222>  (9)..(11)
<223>  1,2-Dideoxy-B-D-ribose-3-CEP

<400>  18
tggcgcacnn ngctgggag                                                     19


<210>  19
<211>  20
<212>  DNA
<213>  Artificial Sequence

<220>
<223>  Artificially synthesized oligonucleotide sequence


<220>
<221>  misc_feature
<222>  (9)..(12)
<223>  1,2-Dideoxy-B-D-ribose-3-CEP

<400>  19
tggcgcacnn nngctgggag                                                    20


<210>  20
<211>  20
<212>  DNA
<213>  Artificial Sequence

<220>
<223>  Artificially synthesized oligonucleotide sequence


<220>
<221>  misc_feature
<222>  (9)..(12)
<223>  "U" at these positions is RNA

<400>  20
tggcgcacuu uugctgggag                                                    20

<210>  21
<211>  16
<212>  DNA
<213>  Artificial Sequence

<220>
<223>  Artificially synthesized oligonucleotide sequence


<220>
<221>  modified_base
<222>  (1)..(3)
<223>  LNA

<220>
<221>  modified_base
<222>  (1)..(16)
<223>  Phosphorothioate bonds between nucleic acids

<220>
<221>  modified_base
<222>  (14)..(16)
<223>  LNA

<400>  21
ctagttcact gaatgc                                                        16


<210>  22
<211>  20
<212>  DNA
<213>  Artificial Sequence

<220>
<223>  Artificially synthesized oligonucleotide sequence


<220>
<221>  misc_feature
<222>  (9)..(12)
<223>  "U" at these positions is RNA

<400>  22
gcattcaguu uutgaactag                                                    20

## Description

### [Technical Field]

The present invention relates to a nucleic acid, preferably a double-stranded nucleic acid, having an activity of suppressing the expression of a target gene by means of, for example, an antisense effect, and more particularly to a nucleic acid, preferably a double-stranded nucleic acid, comprising an antisense nucleic acid complementary to a transcript of a target gene and a nucleic acid complementary to the nucleic acid, the complementary nucleic acid having at least one bulge structure.

### [Background Art]

In recent years, the development of oligonucleotides as pharmaceutical products referred to as nucleic acid drugs has been in progress, and particularly from the viewpoint of high selectivity for target genes and low toxicity, the development of nucleic acid drugs utilizing antisense technologies has been actively in progress. Antisense technologies involve introducing into cells an oligonucleotide (antisense oligonucleotide (ASO)) complementary to a partial sequence of the mRNA (sense strand) of a target gene, thereby selectively inhibiting the expression of the protein encoded by the target gene.

When an oligonucleotide composed of RNA is introduced as an ASO into cells, the ASO binds to a transcript (mRNA) of a target gene to form a partial duplex. It is known that this duplex serves as a cover to prevent translation by ribosomes to inhibit the expression of the protein encoded by the target gene.

On the other hand, when an oligonucleotide composed of DNA is introduced as an ASO into cells, a partial DNA-RNA heteroduplex oligonucleotide is formed. This structure is then recognized by RNase H that degrades the mRNA of the target gene, leading to inhibition of the expression of the protein encoded by the target gene. Furthermore, the use of DNA as an ASO (RNase H-dependent pathway) has been found to typically result in an effect of suppressing gene expression higher than that achieved by using RNA.

In connection with the antisense effect obtained with DNA as an ASO, Patent Literature 1 discloses a double-stranded nucleic acid complex prepared by annealing an LNA/DNA gapmer to a strand composed of RNA that is complementary to the gapmer. Patent Literature 1 describes that the antisense effect of this double-stranded nucleic acid is typically equivalent to that of the single-stranded LNA/DNA gapmer.

### [Citation List]

### [Patent Literature]

[Patent Literature 1] National Publication of International Patent Application No. 2015-502134

### [Summary of Invention]

### [Technical Problem]

An object of the present invention is to provide a nucleic acid complex, preferably a double-stranded nucleic acid complex, having an excellent effect of suppressing the expression of a target gene.

Still another object of the present invention is to provide a nucleic acid complex, preferably a double-stranded nucleic acid complex, which can be delivered to a target site with high specificity and efficiency.

Other objects of the present invention will become apparent from the following description.

### [Solution to Problem]

The present inventors have found that through the use of a nucleic acid complex, preferably a double-stranded nucleic acid complex, which comprises an active moiety comprising an antisense nucleic acid complementary to a transcript, for example, a transcript of a target gene, and a carrier moiety comprising a nucleic acid complementary to the nucleic acid, and having at least one bulge structure, the expression of the transcript can be markedly suppressed.

Thus, the present invention relates to the following:
1. A nucleic acid complex comprising:
   (i) an active moiety comprising a polynucleotide comprising at least one or more deoxyribonucleotides and optionally one or more modified nucleotides and/or nucleotide analogs as structural units; and
   (ii) a carrier moiety comprising a polynucleotide comprising one or more nucleotides and optionally one or more modified nucleotides and/or nucleotide analogs as structural units, the polynucleotide being at least partially complementary to the polynucleotide of (i) the active moiety, wherein
      the polynucleotide of (ii) the carrier moiety comprises at least one bulge.
2. The nucleic acid complex according to 1 above, which is a double-stranded nucleic acid complex.
3. The nucleic acid complex according to 1 or 2 above, wherein the polynucleotide of (ii) the carrier moiety comprises one to three bulges.
4. The nucleic acid complex according to any one of 1 to 3 above, wherein a portion or all of the deoxyribonucleotides of the polynucleotide of (i) the active moiety are modified.
5. The nucleic acid complex according to any one of 1 to 4 above, wherein the polynucleotide of (i) the active moiety comprises one or more modified nucleotides and/or nucleotide analogs.
6. The nucleic acid complex according to any one of 1 to 5 above, wherein the polynucleotide of (i) the active moiety comprises at least four contiguous deoxyribonucleotides, and
   the polynucleotide comprises:
   (a) a 5'-wing region that is positioned 5' to the at least four contiguous deoxyribonucleotides, and comprises one or more modified nucleotides and/or nucleotide analogs; and/or
   (b) a 3'-wing region that is positioned 3' to the at least four contiguous deoxyribonucleotides, and comprises one or more modified nucleotides and/or nucleotide analogs.
7. The nucleic acid complex according to 6 above, wherein the polynucleotide of (i) the active moiety comprises (a) the 5'-wing region and (b) the 3'-wing region, the 5'-wing region comprises at least two modified nucleotides and/or nucleotide analogs, and the 3'-wing region comprises at least two modified nucleotides and/or nucleotide analogs.
8. The nucleic acid complex according to 7 above, wherein the 5'-wing region comprises two to five modified nucleotides and/or nucleotide analogs, and the 3'-wing region comprises two to five modified nucleotides and/or nucleotide analogs.
9. The nucleic acid complex according to any one of 1 to 8 above, wherein the polynucleotide of (i) the active moiety has one or more nucleotide analogs, and at least one of the nucleotide analogs is modified.
10. The nucleic acid complex according to any one of 1 to 9 above, wherein the polynucleotide of (ii) the carrier moiety has one or more nucleotide analogs, and at least one of the nucleotide analogs is modified.
11. The nucleic acid complex according to any one of 6 to 10 above, wherein the at least four contiguous deoxyribonucleotides are phosphorothioated.
12. The nucleic acid complex according to any one of 7 to 11 above, wherein each of the 5'-wing region and the 3'-wing region comprises a modified nucleotide and/or a bridged nucleotide as a nucleotide analog.
13. The nucleic acid complex according to 12 above, wherein the bridged nucleotide is selected from the group consisting of LNA, cEt-BNA, amide BNA (AmNA), and cMOE-BNA.
14. The nucleic acid complex according to 12 or 13 above, wherein the bridged nucleotide is phosphorothioated.
15. The nucleic acid complex according to any one of 1 to 14 above, wherein the polynucleotide of (ii) the carrier moiety comprises at least one mismatch compared to the polynucleotide of (i) the active moiety.
16. The nucleic acid complex according to any one of 1 to 15 above, wherein the polynucleotide of (i) the active moiety is 8 to 100 bases in length.
17. The nucleic acid complex according to any one of 1 to 16 above, wherein the polynucleotide of (ii) the carrier moiety is 8 to 100 bases in length.
18. The nucleic acid complex according to any one of 1 to 17 above, wherein the polynucleotide of (i) the active moiety is identical in length to the polynucleotide of (ii) the carrier moiety when compared with the bulge being excluded from the polynucleotide of (ii) the carrier moiety.
19. The nucleic acid complex according to any one of 1 to 18 above, wherein the polynucleotide of (i) the active moiety is different in length from the polynucleotide of (ii) the carrier moiety when compared with the bulge being excluded from the polynucleotide of (ii) the carrier moiety.
20. The nucleic acid complex according to any one of 1 to 19 above, wherein the polynucleotide of (ii) the carrier moiety comprises one or more deoxyribonucleotides and/or ribonucleotides as the nucleotides forming the structural units.
21. The nucleic acid complex according to any one of 1 to 9 and 11 to 20 above, wherein the polynucleotide of (ii) the carrier moiety is free of a modified nucleotide.
22. The nucleic acid complex according to any one of 1 to 9 and 11 to 21 above, wherein the polynucleotide of (ii) the carrier moiety is free of a nucleotide analog.
23. The nucleic acid complex according to any one of 1 to 22 above, wherein the bulge is formed of one or more deoxyribonucleotides and/or ribonucleotides.
24. The nucleic acid complex according to 23 above, wherein the bulge is formed of one or more ribonucleotides.
25. The nucleic acid complex according to any one of 1 to 24 above, wherein (ii) the carrier moiety further comprises a functional moiety having a function selected from a labeling function, a purification function, and a targeted delivery function.
26. The nucleic acid complex according to 25 above, wherein the functional moiety is a molecule selected from lipids, peptides, and proteins.
27. The nucleic acid complex according to any one of 1 to 26 above, for use in reducing expression of a target gene in a mammal.
28. The nucleic acid complex according to 27 above, wherein the polynucleotide of the active moiety is an antisense strand complementary to any region of mRNA of the target gene.
29. A pharmaceutical composition comprising the nucleic acid complex according to any one of 1 to 28 above and optionally a pharmacologically acceptable carrier.
30. A method for reducing the level of a transcript in a cell comprising the step of contacting the nucleic acid complex according to any one of 1 to 26 above with the cell, wherein
   the polynucleotide of the active moiety is an antisense strand complementary to any region of the transcript.
31. The method according to 30 above, wherein the transcript is a protein-coding mRNA transcript.
32. The method according to 30 above, wherein the transcript is a non-protein-coding transcript.
33. Use of the nucleic acid complex according to any one of 1 to 26 above for reducing expression of a target gene in a mammal.
34. Use of the nucleic acid complex according to any one of 1 to 26 above for the manufacture of a medicament for reducing expression of a target gene in a mammal.
35. A method for reducing the expression level of a target gene in a mammal comprising the step of administering the nucleic acid complex according to any one of 1 to 26 above to a mammal, wherein
   the polynucleotide of the active moiety is an antisense strand complementary to any region of mRNA of the target gene.
36. The method according to 35 above, wherein the administration is enteral administration.
37. The method according to 35 above, wherein the administration is non-enteral administration.
38. The method according to any one of 35 to 37 above, wherein the nucleic acid complex is administered at a dose of 0.001 to 50 mg/kg/day.
39. The method according to any one of 35 to 38 above, wherein the mammal is a human.

The present invention may also encompass the following additional embodiments:
1. A nucleic acid complex comprising:
   (i) an active moiety comprising a polynucleotide comprising at least one or more deoxyribonucleotides and optionally one or more modified nucleotides and/or nucleotide analogs as structural units; and
   (ii) a carrier moiety comprising a polynucleotide comprising one or more nucleotides and optionally one or more modified nucleotides and/or nucleotide analogs as structural units, the polynucleotide being at least partially complementary to the polynucleotide of (i) the active moiety, wherein
      the polynucleotide of (ii) the carrier moiety comprises at least one bulge.
2. The nucleic acid complex according to 1 above, which is a double-stranded nucleic acid complex.
3. The nucleic acid complex according to 1 or 2 above, wherein the polynucleotide of (ii) the carrier moiety comprises one to three bulges.
4. The nucleic acid complex according to 3 above, wherein the polynucleotide of (ii) the carrier moiety comprises one bulge.
5. The nucleic acid complex according to any one of 1 to 4 above, wherein a portion or all of the deoxyribonucleotides of the polynucleotide of (i) the active moiety are modified.
6. The nucleic acid complex according to any one of 1 to 5 above, wherein the polynucleotide of (i) the active moiety comprises one or more modified nucleotides and/or nucleotide analogs.
7. The nucleic acid complex according to any one of 1 to 6 above, wherein the polynucleotide of (i) the active moiety comprises one or more nucleotide analogs, and at least one of the nucleotide analogs is optionally modified.
8. The nucleic acid complex according to any one of 1 to 7 above, wherein the polynucleotide of (i) the active moiety comprises at least four contiguous deoxyribonucleotides, and
   the polynucleotide comprises:
   (a) a 5'-wing region that is positioned 5' to the at least four contiguous deoxyribonucleotides, and comprises one or more modified nucleotides and/or nucleotide analogs; and/or
   (b) a 3'-wing region that is positioned 3' to the at least four contiguous deoxyribonucleotides, and comprises one or more modified nucleotides and/or nucleotide analogs.
9. The nucleic acid complex according to 8 above, wherein the polynucleotide of (i) the active moiety comprises (a) the 5'-wing region and (b) the 3'-wing region.
10. The nucleic acid complex according to 8 or 9 above, wherein the 5'-wing region comprises two to five modified nucleotides and/or nucleotide analogs, and the 3'-wing region comprises two to five modified nucleotides and/or nucleotide analogs.
11. The nucleic acid complex according to 8 or 9 above, wherein the 5'-wing region comprises one modified nucleotide and/or nucleotide analog, and the 3'-wing region comprises one modified nucleotide and/or nucleotide analog.
12. The nucleic acid complex according to any one of 8 to 11 above, wherein each of the 5'-wing region and the 3'-wing region comprises a sugar-modified nucleotide and/or a bridged nucleotide as a nucleotide analog.
13. The nucleic acid complex according to 12 above, wherein the bridged nucleotide is selected from the group consisting of LNA, cEt-BNA, amide BNA (AmNA), and cMOE-BNA.
14. The nucleic acid complex according to 13 above, wherein the bridged nucleotide is phosphorothioated.
15. The nucleic acid complex according to any one of 8 to 14 above, wherein the at least four contiguous deoxyribonucleotides are phosphorothioated.
16. The nucleic acid complex according to any one of 8 to 15 above, wherein the polynucleotide of (i) the active moiety consists of a 5'-wing region consisting of one or more phosphorothioated bridged nucleotides, phosphorothioated deoxyribonucleotides, and a 3'-wing region consisting of one or more phosphorothioated bridged nucleotides.
17. The nucleic acid complex according to 8 or 9 above, wherein the 5'-wing region consists of one to five sugar-modified ribonucleotides, and the 3'-wing region consists of one to five sugar-modified ribonucleotides, and wherein the sugar-modified ribonucleotides are optionally phosphorothioated.
18. The nucleic acid complex according to 17 above, wherein the polynucleotide of (i) the active moiety consists of a 5'-wing region consisting of one or more phosphorothioated sugar-modified ribonucleotides, at least four contiguous phosphorothioated deoxyribonucleotides, and a 3'-wing region consisting of one or more phosphorothioated sugar-modified ribonucleotides.
19. The nucleic acid complex according to 17 or 18 above, wherein the sugar modification is selected from the group consisting of 2'-O-methylation, 2'-O-methoxyethylation (2'-MOE modification), 2'-O-aminopropylation (2'-AP modification), and 2'-fluorination.
20. The nucleic acid complex according to any one of 1 to 5 above, wherein the polynucleotide of (i) the active moiety consists of at least four contiguous phosphorothioated deoxyribonucleotides.
21. The nucleic acid complex according to any one of 1 to 20 above, wherein the polynucleotide of (ii) the carrier moiety comprises one or more deoxyribonucleotides as structural units.
22. The nucleic acid complex according to 21 above, wherein the polynucleotide of (ii) the carrier moiety consists of one or more deoxyribonucleotides.
23. The nucleic acid complex according to any one of 1 to 21 above, wherein the polynucleotide of (ii) the carrier moiety comprises one or more ribonucleotides as structural units.
24. The nucleic acid complex according to 21 or 23 above, wherein the polynucleotide of (ii) the carrier moiety consists of one or more ribonucleotides and one or more deoxyribonucleotides.
25. The nucleic acid complex according to 23 or 24 above, wherein the ribonucleotides are unmodified.
26. The nucleic acid complex according to any one of 21 to 25 above, wherein the deoxyribonucleotides are unmodified.
27. The nucleic acid complex according to any one of 21 to 25 above, wherein a portion or all of the deoxyribonucleotides are modified.
28. The nucleic acid complex according to 27 above, wherein the modified deoxyribonucleotides are base-modified deoxyribonucleotides.
29. The nucleic acid complex according to 28 above, wherein the base-modified deoxyribonucleotides are abasic deoxyribonucleotides.
30. The nucleic acid complex according to any one of 27 to 29 above, wherein the modified deoxyribonucleotides have no modifications other than the base modification.
31. The nucleic acid complex according to any one of 1 to 30 above, wherein the bulge is formed of one or more deoxyribonucleotides and/or ribonucleotides.
32. The nucleic acid complex according to 31 above, wherein the bulge is formed of one or more deoxyribonucleotides.
33. The nucleic acid complex according to 32 above, wherein the bulge is formed of one or more unmodified deoxyribonucleotides.
34. The nucleic acid complex according to 32 above, wherein the bulge is formed of one or more base-modified deoxyribonucleotides.
35. The nucleic acid complex according to 31 above, wherein the bulge is formed of one or more ribonucleotides.
36. The nucleic acid complex according to 35 above, wherein the bulge is formed of one or more unmodified ribonucleotides.
37. The nucleic acid complex according to any one of 1 to 36 above, wherein the polynucleotide of (i) the active moiety comprises at least one mismatch compared to the polynucleotide of (ii) the carrier moiety and/or a target transcript.
38. The nucleic acid complex according to any one of 1 to 36 above, wherein the polynucleotide of (i) the active moiety comprises no mismatch compared to the polynucleotide of (ii) the carrier moiety and/or a target transcript.
39. The nucleic acid complex according to any one of 1 to 38 above, wherein the polynucleotide of (ii) the carrier moiety comprises at least one mismatch compared to the polynucleotide of (i) the active moiety.
40. The nucleic acid complex according to any one of 1 to 38 above, wherein the polynucleotide of (ii) the carrier moiety comprises no mismatch compared to the polynucleotide of (i) the active moiety.
41. The nucleic acid complex according to any one of 1 to 40 above, wherein the polynucleotide of (i) the active moiety is 8 to 100 bases in length.
42. The nucleic acid complex according to any one of 1 to 41 above, wherein the polynucleotide of (ii) the carrier moiety is 8 to 100 bases in length.
43. The nucleic acid complex according to any one of 1 to 42 above, wherein the polynucleotide of (i) the active moiety is identical in length to the polynucleotide of (ii) the carrier moiety when compared with the bulge being excluded from the polynucleotide of (ii) the carrier moiety.
44. The nucleic acid complex according to any one of 1 to 42 above, wherein the polynucleotide of (i) the active moiety is different in length from the polynucleotide of (ii) the carrier moiety when compared with the bulge being excluded from the polynucleotide of (ii) the carrier moiety.
45. The nucleic acid complex according to any one of 1 to 44 above, wherein (ii) the carrier moiety further comprises a functional moiety having a function selected from a labeling function, a purification function, and a targeted delivery function.
46. The nucleic acid complex according to 45 above, wherein (ii) the carrier moiety comprises the functional moiety having the targeted delivery function, and the functional moiety is a molecule selected from the group consisting of lipids, peptides, proteins, sugar chains, small molecules, and biomolecules/bioactive molecules.
47. The nucleic acid complex according to 46 above, wherein the functional moiety is a peptide containing a cyclic arginine-glycine-aspartic acid (RGD) sequence, N-acetylgalactosamine, cholesterol, vitamin E (tocopherol), stearic acid, docosanoic acid, anisamide, folic acid, anandamide, or spermine.
48. The nucleic acid complex according to any one of 1 to 44 above, wherein (ii) the carrier moiety does not comprise a functional moiety having a targeted delivery function.
49. A double-stranded nucleic acid complex comprising:
   (i) an active moiety comprising a polynucleotide comprising, as structural units, at least four contiguous phosphorothioated deoxyribonucleotides, a 5'-wing region that is positioned 5' to the at least four contiguous deoxyribonucleotides, and consists of two to five phosphorothioated bridged nucleotides, and a 3'-wing region that is positioned 3' to the at least four contiguous deoxyribonucleotides, and consists of two to five phosphorothioated bridged nucleotides; and
   (ii) a carrier moiety comprising a polynucleotide consisting of one or more unmodified ribonucleotides and one or more unmodified deoxyribonucleotides as structural units, the polynucleotide being at least partially complementary to the polynucleotide of (i) the active moiety, wherein
      the polynucleotide of (ii) the carrier moiety comprises at least one bulge, and the bulge is formed of one or more unmodified ribonucleotides,
      the polynucleotide of (i) the active moiety comprises no mismatch compared to the polynucleotide of (ii) the carrier moiety and/or a target transcript, and
   (ii) the carrier moiety does not comprise a functional moiety having a targeted delivery function.
50. The nucleic acid complex according to any one of 1 to 49 above, for use in reducing expression of a target gene in a mammal.
51. The nucleic acid complex according to 50 above, wherein the polynucleotide of the active moiety is an antisense strand complementary to any region of mRNA of the target gene.
52. The nucleic acid complex according to any one of 1 to 49 above, for use in reducing expression of a non-protein-coding transcript in a mammal.
53. The nucleic acid complex according to 52 above, wherein the polynucleotide of the active moiety is an antisense strand complementary to any region of the transcript.
54. A pharmaceutical composition comprising the nucleic acid complex according to any one of 1 to 49 above and optionally a pharmacologically acceptable carrier.
55. A method for reducing the level of a transcript in a cell comprising the step of contacting the nucleic acid complex according to any one of 1 to 49 with the cell, wherein
   the polynucleotide of the active moiety is an antisense strand complementary to any region of the transcript.
56. The method according to 55 above, wherein the transcript is a protein-coding mRNA transcript.
57. The method according to 55 above, wherein the transcript is a non-protein-coding transcript.
58. Use of the nucleic acid complex according to any one of 1 to 49 above for reducing expression of a target gene in a mammal.
59. Use of the nucleic acid complex according to any one of 1 to 49 above for the manufacture of a medicament for reducing expression of a target gene in a mammal.
60. A method for reducing the expression level of a target gene in a mammal comprising the step of administering the nucleic acid complex according to any one of 1 to 49 to a mammal, wherein
   the polynucleotide of the active moiety is an antisense strand complementary to any region of mRNA of the target gene.
61. The method according to 60 above, wherein the administration is enteral administration.
62. The method according to 60 above, wherein the administration is non-enteral administration.
63. The method according to any one of 60 to 62 above, wherein the nucleic acid complex is administered at a dose of 0.001 to 50 mg/kg/day.
64. The method according to any one of 60 to 63 above, wherein the mammal is a human.

### [Advantageous Effects of Invention]

Through the use of the nucleic acid complex with at least one bulge structure, preferably the double-stranded nucleic acid complex, of the present invention, the expression of a target transcript, typically a transcript of a target gene or a non-target-coding RNA, can be suppressed very specifically and efficiently. Furthermore, when the nucleic acid complex has a functional moiety bound thereto, it can be delivered to a target site in the body very selectively and efficiently.

In the present invention, it has been found that, by introducing at least one bulge structure into a double-stranded nucleic acid complex comprising a DNA-based nucleic acid and a nucleic acid complementary to the nucleic acid, the double-stranded nucleic acid complex can achieve a superior antisense effect, and, for example, exhibits a target RNA expression inhibitory effect much superior to that achieved by an ASO having a single-stranded structure in an animal tissue, as demonstrated in the Examples.

### [Brief Description of Drawings]

[Figure 1] Figure 1 shows the results of cell proliferation-suppressing activity tests on human BCL2 gene-targeted double-stranded nucleic acids, using A431 cell line.
[Figure 2] Figure 2 shows the results of cell proliferation-suppressing activity tests on human BCL2 gene-targeted double-stranded nucleic acids, using AsPC-1 cell line.
[Figure 3] Figure 3 shows the results of cell proliferation-suppressing activity tests on human BCL2 gene-targeted double-stranded nucleic acids, using A549 cell line.
[Figure 4] Figure 4 shows the results of cell proliferation-suppressing activity tests on human BCL2 gene-targeted double-stranded nucleic acids, using HCT116 cell line.
[Figure 5] Figure 5 shows the result of an mRNA expression-suppressing activity test on a human BCL2 gene-targeted double-stranded nucleic acid, using A431 cell line.
[Figure 6] Figure 6 shows the results of an mRNA expression-suppressing activity tests on human BCL2 gene-targeted double-stranded nucleic acids, using AsPC-1 cell line.
[Figure 7] Figure 7 shows the results of an mRNA expression-suppressing activity tests on human BCL2 gene-targeted double-stranded nucleic acids, using A549 cell line.
[Figure 8] Figure 8 shows the results of an mRNA expression-suppressing activity tests on human BCL2 gene-targeted double-stranded nucleic acids, using HCT116 cell line.
[Figure 9] Figure 9 shows the results of cell proliferation-suppressing activity tests on human BCL2 gene-targeted double-stranded nucleic acids, using PANC-1 cell line.
[Figure 10] Figure 10 shows the results of cell proliferation-suppressing activity tests on human BCL2 gene-targeted double-stranded nucleic acids, using Capan-1 cell line.
[Figure 11] Figure 11 shows the results of cell proliferation-suppressing activity tests on human BCL2 gene-targeted double-stranded nucleic acids, using Ls174T cell line.
[Figure 12] Figure 12 shows the results of cell proliferation-suppressing activity tests on human BCL2 gene-targeted double-stranded nucleic acids, using HPAC cell line.
[Figure 13] Figure 13 shows the results of cell proliferation-suppressing activity tests on human BCL2 gene-targeted double-stranded nucleic acids, using AsPC-1 cell line.
[Figure 14] Figure 14 shows the results of cell proliferation-suppressing activity tests on human BCL2 gene-targeted double-stranded nucleic acids, using HCT116 cell line.
[Figure 15] Figure 15 shows the results of cell proliferation-suppressing activity tests on human BCL2 gene-targeted double-stranded nucleic acids, using A549 cell line.
[Figure 16] Figure 16 shows the results of cell proliferation-suppressing activity tests on human BCL2 gene-targeted double-stranded nucleic acids, using A431 cell line.
[Figure 17] Figure 17 shows the results of cell proliferation-suppressing activity tests on human BCL2 gene-targeted double-stranded nucleic acids, using PANC-1 cell line.
[Figure 18] Figure 18 shows the results of cell proliferation-suppressing activity tests on human BCL2 gene-targeted double-stranded nucleic acids, using A549 cell line.
[Figure 19] Figure 19 shows the results of cell proliferation-suppressing activity tests on human BCL2 gene-targeted double-stranded nucleic acids, using A431 cell line.
[Figure 20] Figure 20 shows the results of cell proliferation-suppressing activity tests on human BCL2 gene-targeted double-stranded nucleic acids, using AsPC-1 cell line.
[Figure 21] Figure 21 shows the results of cell proliferation-suppressing activity tests on human BCL2 gene-targeted double-stranded nucleic acids, using HCT116 cell line.
[Figure 22] Figure 22 shows the results of cell proliferation-suppressing activity tests on human BCL2 gene-targeted double-stranded nucleic acids, using Caki-1 cell line.
[Figure 23] Figure 23 shows the results of cell proliferation-suppressing activity tests on human BCL2 gene-targeted double-stranded nucleic acids, using MCF-7 cell line.
[Figure 24] Figure 24 shows the results of cell proliferation-suppressing activity tests on human BCL2 gene-targeted double-stranded nucleic acids, using DU145 cell line.
[Figure 25] Figure 25 shows the results of cell proliferation-suppressing activity tests on human BCL2 gene-targeted double-stranded nucleic acids, using LNCaP cell line.
[Figure 26] Figure 26 shows the results of cell proliferation-suppressing activity tests on human BCL2 gene-targeted double-stranded nucleic acids, using PC-3 cell line.
[Figure 27] Figure 27 shows the results of cell proliferation-suppressing activity tests on human BCL2 gene-targeted double-stranded nucleic acids, using HGC 27 cell line.
[Figure 28] Figure 28 shows the results of cell proliferation-suppressing activity tests on human BCL2 gene-targeted double-stranded nucleic acids, using MKN-45 cells.
[Figure 29] Figure 29 shows the results of cell proliferation-suppressing activity tests on human BCL2 gene-targeted double-stranded nucleic acids, using OVCAR-3 cells.
[Figure 30] Figure 30 shows the results of cell proliferation-suppressing activity tests on human BCL2 gene-targeted double-stranded nucleic acids, using HepG2 cells.
[Figure 31] Figure 31 shows the results of cell proliferation-suppressing activity tests on human BCL2 gene-targeted double-stranded nucleic acids, using T24 cells.
[Figure 32] Figure 32 shows the result of an mRNA expression-suppressing activity test on a human BCL2 gene-targeted double-stranded nucleic acid, using A549 cell line.
[Figure 33] Figure 33 shows the results of evaluation of the efficacy of a double-stranded nucleic acid having a basic backbone of DNA-bulge in pancreatic carcinoma cell line-derived liver metastasis mouse models.
[Figure 34] Figure 34 shows the result of verification of a MALAT1 gene knockdown effect in mouse livers of a double-stranded nucleic acid having a basic backbone of DNA-bulge structure.

### [Description of Embodiments]

As described above, the nucleic acid complex of the present invention is a nucleic acid complex, preferably a double-stranded nucleic acid complex, which comprises (i) an active moiety comprising an antisense nucleic acid (DNA-based nucleic acid) and (ii) a carrier moiety comprising a nucleic acid having at least one bulge structure, and at least partially complementary to the antisense nucleic acid. In one embodiment of the present invention, the nucleic acid complex is a purified or isolated nucleic acid complex, preferably a purified or isolated double-stranded nucleic acid complex. The nucleic acid complex has an activity of suppressing the expression of a target gene or the level of a transcript (a protein-coding mRNA transcript or non-protein-coding transcript) by means of an antisense effect.

As used herein, the "active moiety" refers to one of the constituents of the nucleic acid complex, which is a moiety that is believed to be mainly responsible for the function of achieving a principal intended effect concerning the nucleic acid complex, i.e., the effect of suppressing the expression of a target gene or a target transcript (both will also be collectively referred to as a "target transcript", hereinafter). In other words, the active moiety is a moiety having an activity of suppressing the expression of a target transcript.

The active moiety comprises a polynucleotide as an antisense nucleic acid to a target transcript. In one embodiment of the present invention, the active moiety is a polynucleotide, i.e., consists of a polynucleotide only.

The polynucleotide of the active moiety comprises at least one or more deoxyribonucleotides and optionally one or more modified nucleotides and/or nucleotide analogs as structural units. In one embodiment of the present invention, the polynucleotide of the active moiety contains only one or more deoxyribonucleotides and optionally one or more modified nucleotides and/or nucleotide analogs as structural units.

As used herein, the "carrier moiety" refers to one of the constituents of the nucleic acid complex, which is a moiety that is believed to have a function as a carrier of the active moiety for an appropriate period of time until the active moiety reaches a target transcript.

The carrier moiety comprises a polynucleotide that is at least partially complementary to the polynucleotide of the active moiety. Because of this complementarity, the polynucleotide of the carrier moiety forms a duplex with the polynucleotide of the active moiety, thereby serving as a carrier. In one embodiment of the present invention, the carrier moiety is a polynucleotide, i.e., consists of a polynucleotide only.

The polynucleotide of the carrier moiety comprises at least one or more nucleotides and optionally one or more modified nucleotides and/or nucleotide analogs as structural units. In one embodiment of the present invention, the polynucleotide of the carrier moiety contains only one or more nucleotides and optionally one or more modified nucleotides and/or nucleotide analogs as structural units.

The nucleic acid complex may comprise additional moieties (for example, a functional moiety) other than the active moiety and the carrier moiety, in accordance with a function desired in the nucleic acid complex. The nucleic acid complex can comprise such an additional moiety as an independent moiety separate from the active moiety and the carrier moiety, or in a form incorporated into the active moiety or the carrier moiety.

In one embodiment, the nucleic acid complex consists of the active moiety and the carrier moiety only.

The "antisense effect" refers to the suppression of the expression of a target gene or the level of a target transcript, which is induced by the hybridization of the target transcript (RNA sense strand) with, for example, a DNA strand, or generally a strand designed to produce the antisense effect, that is complementary to a partial sequence of the transcript. In certain instances, the above-defined suppression may refer to suppression attributable to the inhibition of translation or a splicing function-modifying effect such as exon skipping that may be induced by covering of the transcript with the hybridization product, and/or degradation of the transcript that may be induced by recognition of the hybridized portion.

The "target gene" or "target transcript" whose expression is suppressed by the antisense effect is not particularly limited, and examples thereof include genes whose expression is increased in various diseases. Moreover, the "transcript of a target gene" refers to mRNA transcribed from the genomic DNA encoding the target gene, and also includes, for example, mRNA without base modifications and unspliced mRNA precursors. In general, the "transcript" may be any RNA that is synthesized with a DNA-dependent RNA polymerase, and includes non-coding RNAs, for example.

The term "purified or isolated nucleic acid complex" herein refers to a nucleic acid complex comprising at least two polynucleotide strands, which does not occur in nature, and/or is substantially free of naturally occurring nucleic acid materials.

In one embodiment of the present invention, the nucleic acid complex is a double-stranded nucleic acid complex.

The term "complementary" refers to the relationship between one nucleotide base and another such that they can form a so-called Watson-Crick base pair (natural base pair) or a non-Watson-Crick base pair (Hoogsteen base pair, for example) via hydrogen bonding. For example, adenine (A) is complementary to thymidine (T) in DNA, and adenine (A) is complementary to uracil (U) in RNA.

For example, if a nucleotide at a certain position of the polynucleotide of the active moiety is capable of base pairing with a nucleotide at a certain position of a transcript of a target gene via hydrogen bonding, then the polynucleotide and the transcript are considered to be complementary at the position of hydrogen bonding.

In order for a certain polynucleotide to be hybridized or annealed (these terms are used interchangeably herein) to another polynucleotide, the two polynucleotides need not be complementary to each other at all the positions of their base sequences. Likewise, in the context of the present invention, in order for the nucleic acid complex to exhibit its functions, the polynucleotides need not be complementary to each other at all the positions of their base sequences, and may include mismatches at some positions.

For example, the base sequence of the target transcript need not be fully complementary to the base sequence of the polynucleotide of (i) the active moiety, i.e., the two base sequences need not be complementary to each other at all the positions.

In the present invention, the polynucleotide of the active moiety is at least partially complementary to a target transcript. In one embodiment of the present invention, the base sequence of the target transcript and the base sequence of the polynucleotide of the active moiety may be at least 70% or more, preferably 80% or more, and more preferably 90% or more (for example, 95%, 96%, 97%, 98%, 99% or more, or 100%) complementary to each other. If 70% of the nucleotides are complementary, the two base sequences will have 70% "complementarity". In one preferred embodiment of the present invention, the two base sequences are fully complementary, i.e., have 100% complementarity.

The complementarity of two polynucleotides (for example, complementarity between a target transcript and the polynucleotide of the active moiety) may be calculated as the complementarity in the duplex-forming region (or the entire duplex-forming region including mismatches, if any), if these polynucleotides have different lengths.

The sequence complementarity may be determined using a BLAST program, for example. A person skilled in the art can readily design an antisense nucleic acid complementary to a target transcript on the basis of, for example, information of the base sequence of the target gene.

In the present invention, the polynucleotide of the active moiety and the polynucleotide of the carrier moiety can form a duplex by being "annealed" on the basis of the above-described complementarity. A person skilled in the art can readily determine the conditions (temperature, salt concentration, etc.) under which the two nucleic acid strands can be annealed.

As used herein, the "nucleic acid" may refer to a monomeric nucleotide, or may refer to an oligonucleotide or polynucleotide composed of a plurality of monomers. The term "nucleic acid strand" is also used herein to collectively refer to an oligonucleotide and a polynucleotide. Nucleic acid strands may be prepared completely or partially by chemical synthesis methods including the use of an automated synthesizer, or by enzymatic treatment including, although not limited to, polymerase, ligase, and restriction enzyme reactions.

In the present invention, the polynucleotide of the active moiety comprises at least "one or more deoxyribonucleotides and optionally one or more modified nucleotides and/or nucleotide analogs" as structural units. This phrase is intended to mean that the polynucleotide has one or more deoxyribonucleotides, and may further optionally have one or more modified nucleotides and/or nucleotide analogs.

The polynucleotide of the carrier moiety comprises "one or more nucleotides and optionally one or more modified nucleotides and/or nucleotide analogs" as structural units. This phrase is intended to mean that the polynucleotide has one or more nucleotides, and may further optionally have one or more modified nucleotides and/or nucleotide analogs. The nucleotides as essential structural units herein may be deoxyribonucleotides, ribonucleotides, or a combination thereof. Each of the nucleotides may be independently modified or unmodified.

The polynucleotide of the active moiety is not particularly limited in length, and is preferably at least 8 bases, at least 10 bases, at least 12 bases, at least 13 bases, at least 14 bases, or at least 15 bases in length. The length may be preferably 100 bases or less, 35 bases or less, 25 bases or less, 20 bases or less, 19 bases or less, 18 bases or less, or 17 bases or less. The range of lengths is preferably from 10 to 35 bases, more preferably from 12 to 25 bases, and still more preferably from 13 to 20 bases. In general, the length is selected according to the strength of the antisense effect of the nucleic acid strand against the target, as well as other factors such as cost and synthesis yield.

In one embodiment of the present invention, the polynucleotide of the active moiety may be an antisense polynucleotide that is at least partially complementary to a target transcript, which polynucleotide has a region comprising at least four contiguous deoxyribonucleotides.

The "at least four contiguous deoxyribonucleotides" can be a region comprising 4 to 20 contiguous deoxyribonucleotides, preferably a region comprising 5 to 16 contiguous deoxyribonucleotides, and more preferably 6 to 14, for example, 7 to 13, contiguous deoxyribonucleotides. The nucleotides to be used in this region may be nucleotides such as natural DNAs that are recognized by RNase H that cleaves an RNA strand, upon hybridization to ribonucleotides. In one embodiment of the present invention, each of the deoxyribonucleotides may be modified independently of one another. Modifications applicable to deoxyribonucleotides are known in the art.

As used herein, the "nucleotide" refers to a compound in which the sugar moiety of a nucleoside forms an ester with phosphoric acid, wherein the "nucleoside" refers to a glycoside compound in which a nitrogen-containing organic base such as a purine base or pyrimidine base is linked to the reducing group of a sugar via a glycosidic linkage.

The "deoxyribonucleotide" is a nucleotide in which the sugar moiety is D-2-deoxyribose, and the "ribonucleotide" refers to a nucleotide in which the sugar moiety is D-ribose.

The "polynucleotide" refers to a chain-like substance that is a polymer of a plurality of nucleotides as basic units, wherein adjacent nucleotides are bridged by a diester linkage formed by phosphoric acid between the 3' and 5' carbon atoms of the respective sugars. This term also includes oligonucleotides. The nucleotides contained in the polynucleotide as structural units (in a polymerized state) are also similarly referred to as "nucleotides" herein.

The "deoxyribonucleotide" mentioned hereinabove refers to a naturally occurring deoxyribonucleotide, or a deoxyribonucleotide with a modified base, sugar, or phosphate linkage subunit. Likewise, the "ribonucleotide" refers to a naturally occurring ribonucleotide, or a ribonucleotide with a modified base, sugar, or phosphate linkage subunit. The modification of a base, sugar, or phosphate linkage subunit refers to the addition of a single substituent, or a single substitution within the subunit, and does not refer to the substitution of the entire subunit with a different chemical group. From the viewpoint of having high resistance to a DNase, for example, a portion or all of the region consisting of deoxyribonucleotides may be formed of modified nucleotide(s). Examples of such modifications include 5-methylation, 5-fluorination, 5-bromination, 5-iodination, and N4-methylation of cytosine; 5-demethylation, 5-fluorination, 5-bromination, and 5-iodination of thymidine; N6-methylation and 8-bromination of adenine; N2-methylation and 8-bromination of guanine; phosphorothioation, methylphosphonation, methylthiophosphonation, chiral methylphosphonation, phosphorodithioation, phosphoroamidation, 2'-O-methylation, 2'-methoxyethylation (MOE modification), 2'-aminopropylation (AP modification), and 2'-fluorination. From the viewpoint of having excellent pharmacokinetics, phosphorothioation (thiophosphate linkage) is preferred. A phosphorothioate linkage has a structure wherein one of the oxygen atoms of a phosphate group is substituted with a sulfur atom in a phosphodiester linkage that is a linkage between natural nucleic acids, and is represented by the following formula: 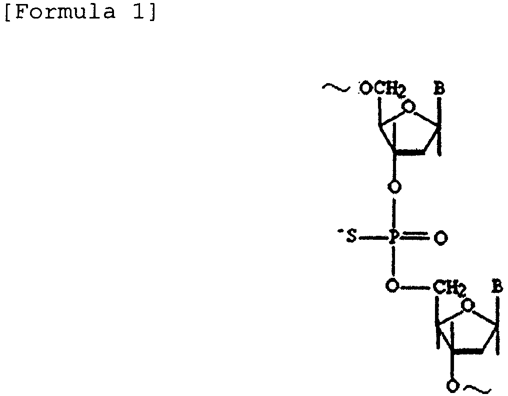 wherein B represents a base.

Furthermore, in order to achieve a similar effect, when the polynucleotide comprises one or more ribonucleotides in the present invention, the ribonucleotides may be similarly modified. In general, naturally occurring ribonucleotides/deoxyribonucleotides correspond to RNA in which ribonucleotides having any base of adenine, guanine, cytosine, and uracil are linked, or DNA in which deoxyribonucleotides having any base of adenine, guanine, cytosine, and thymine are linked. In the present invention, however, ribonucleotides/deoxyribonucleotides may have bases other than the above-mentioned bases, for example, inosine and deoxyinosine.

In one embodiment of the present invention, therefore, the polynucleotide of the active moiety may comprise at least one, preferably one to six, for example, one, ribonucleotide having inosine as a base or deoxyribonucleotide having deoxyinosine as a base. Likewise, the polynucleotide of the carrier moiety may comprise at least one, preferably one to six, for example, three to five, ribonucleotides having inosine as a base or deoxyribonucleotides having deoxyinosine as a base.

In a further embodiment of the present invention, the polynucleotide of the active moiety is free of a ribonucleotide having inosine as a base or a deoxyribonucleotide having deoxyinosine as a base. In an alternative embodiment of the present invention, the polynucleotide of the carrier moiety is free of a ribonucleotide having inosine as a base or a deoxyribonucleotide having deoxyinosine as a base.

A person skilled in the art can select, in accordance with the type of the nucleotide (for example, a deoxyribonucleotide or a ribonucleotide), a possible modification for each nucleotide, and modify the nucleotide, as appropriate.

Furthermore, a single nucleotide may have a combination of the above-described modifications.

In general, the above-described modifications have no effect on base pairing, and hence, are believed to have no effect on the complementarity between nucleotides or complementarity between polynucleotides. That is, two complementary nucleotides can generally maintain the base-pairing capability even after modification, and thus, can remain complementary.

In certain instances, however, the number or positions of modifications may affect the antisense effect and the like to be achieved by the double stranded nucleic acid disclosed herein. These embodiments cannot be unequivocally stated since they may vary with the sequence of a target gene and the like; however, a person skilled in the art can determine them by referring to the teachings of documents concerning antisense technologies. Furthermore, a modification can be evaluated as follows: the antisense effect of a modified nucleic acid complex is measured, and if the measured value is not significantly lower than that of the nucleic acid complex before being modified (for example, if the measured value of the modified nucleic acid complex is 30% or more of the measured value of the nucleic acid complex before being modified), then the modification can be evaluated as a preferred one. The antisense effect can be measured, for example, as described in the Examples below, by introducing a test nucleic acid compound into cells or the like, and then measuring the expression level of a target gene (level of mRNA, cDNA, protein, or the like) in the cells or the like in which the expression is suppressed by the antisense effect achieved by the test nucleic acid compound, using a known technique such as Northern Blotting, quantitative PCR, or Western Blotting, as appropriate.

As used herein, the "nucleotide analog" refers to a non-naturally occurring nucleotide, wherein the base, sugar, or phosphate linkage subunit has two or more substituents added or has two or more substitutions, or the entire subunit is substituted with a different chemical group. An example of an analog with two or more substitutions is a bridged nucleic acid. A bridged nucleic acid is a nucleotide analog in which a bridging unit has been added as a result of two substitutions on the sugar ring, typically a nucleotide analog in which the 2' and 4' carbon atoms are linked. The polynucleotide of the active moiety in one embodiment further comprises a nucleotide analog, from the viewpoint of enhancing the affinity for a partial sequence of a transcript of a target gene and/or the nuclease resistance. The "nucleotide analog" may be any nucleotide that meets the above-described definition, and has enhanced affinity for a partial sequence of a transcript of a target gene and/or enhanced nuclease resistance attributable to the modification (bridging, substitution, etc.). Examples of such nucleotide analogs include the nucleic acids disclosed as being suitable for use in antisense technologies in, for example, Japanese Patent Laid-Open No. 10-304889, WO 2005/021570, Japanese Patent Laid-Open No. 10-195098, National Publication of International Patent Application No. 2002-521310, WO 2007/143315, WO 2008/043753, WO 2008/029619, and WO 2008/049085 (these documents will also be referred to as "documents concerning antisense technologies", hereinafter). Examples of the nucleic acids disclosed in these documents include a hexitol nucleic acid (HNA), a cyclohexane nucleic acid (CeNA), a peptide nucleic acid (PNA), a glycol nucleic acid (GNA), a threose nucleic acid (TNA), a morpholino nucleic acid, a tricyclo-DNA (tcDNA), a 2'-O-methylated nucleic acid, a 2'-MOE modified (2'-O-methoxyethylated) nucleic acid, a 2'-AP modified (2'-O-aminopropylated) nucleic acid, a 2'-fluorinated nucleic acid, a 2'-F-arabinonucleic acid (2'-F-ANA), and a BNA (bridged nucleic acid). These nucleic acids may also be referred to herein as "modifications" or "modified nucleotides".

As a BNA (also referred to herein as a "bridged nucleotide"), for example, a ribonucleotide or a deoxyribonucleotide in which the 2' and 4' carbon atoms are bridged by two or more atoms can be used. Examples of bridged nucleic acids are known to those skilled in the art. One subgroup of such BNAs include BNAs wherein the 2' and 4' carbon atoms are bridged by 4'-(CH₂)ₚ-O-2'; 4'-(CH₂)ₚ-S-2'; 4'-(CH₂)ₚ-OCO-2'; and 4'-(CH₂)ₙ-N(R³)-O-(CH₂)ₘ-2'; wherein p is an integer from 1 to 4, m is an integer from 0 to 2, and n is an integer from 1 to 3; and R³ represents a hydrogen atom, an alkyl group, an alkenyl group, a cycloalkyl group, an aryl group, an aralkyl group, an acyl group, a sulfonyl group, or a unit substituent (for example, a fluorescent or chemiluminescent labeling molecule, a functional group with nucleic acid-cleaving activity, or an intracellular or intranuclear localization signal peptide). Furthermore, in such a BNA in one further embodiment of the present invention, R¹ and R² in OR² as a substituent on the 3' carbon atom and OR¹ as a substituent on the 5' carbon atom may be the same or different, although they are typically a hydrogen atom, and may be a hydroxy-protecting group for nucleic acid synthesis, an alkyl group, an alkenyl group, a cycloalkyl group, an aryl group, an aralkyl group, an acyl group, a sulfonyl group, a silyl group, a phosphoric acid group, a phosphoric acid group protected by a protecting group for nucleic acid synthesis, or -P(R⁴)R⁵; wherein R⁴ and R⁵ may be the same or different, and each represent a hydroxyl group, a hydroxyl group protected by a protecting group for nucleic acid synthesis, a mercapto group, a mercapto group protected by a protecting group for nucleic acid synthesis, an amino group, a C₁₋₅ alkoxy group, a C₁₋₅ alkylthio group, a C₁₋₆ cyanoalkoxy group, or an amino group substituted with a C₁₋₅ alkyl group. Examples of such BNAs include α-L-methyleneoxy (4'-CH₂-O-2') BNA or β-D-methyleneoxy (4'-CH₂-O-2') BNA, also referred to as an LNA (Locked Nucleic Acid (registered trademark); 2',4'-BNA); ethyleneoxy (4'-(CH₂)₂-O-2') BNA, also referred to as an ENA; β-D-thio (4'-CH₂-S-2') BNA; aminooxy (4'-CH₂-O-N(R³)-2') BNA; oxyimino (4'-CH₂-N(R³)-O-2') BNA, also referred to as 2',4'-BNANC; 2',4'-BNACOC; 3'-amino-2',4'-BNA; 5'-methyl BNA; (4'-CH(CH₃)-O-2') BNA, also referred to as cEt-BNA; (4'-CH(CH₂OCH₃)-O-2') BNA, also referred to as cMOE-BNA; amide BNA (4'-C(O)-N(R)-2') BNA (R = H, Me), also referred to as AmNA; and other BNAs known to those skilled in the art.

In one embodiment of the present invention, the modified nucleotide may have a modified base moiety. Examples of modifications in the base moiety include 5-methylation, 5-fluorination, 5-bromination, 5-iodination, and N4-methylation of cytosine; 5-demethylation, 5-fluorination, 5-bromination, and 5-iodination of thymidine; N6-methylation and 8-bromination of adenine; and N2-methylation and 8-bromination of guanine. Furthermore, in the modified nucleotide in a certain embodiment, the phosphodiester binding site may be modified. Examples of modifications of the phosphodiester binding site include phosphorothioation, methylphosphonation, methylthiophosphonation, chiral methylphosphonation, phosphorodithioation, and phosphoroamidation. From the viewpoint of having excellent pharmacokinetics, phosphorothioation is preferred. Furthermore, a single nucleotide may have a combination of the above-described base moiety modifications or phosphodiester binding site modifications.

In one embodiment of the present invention, the nucleotide analog may have a modification (or a combination of modifications) as described above for the modification of the deoxyribonucleotides.

A person skilled in the art can select, in accordance with the type of the nucleotide analog, a possible modification for each nucleotide analog, and modify the nucleotide analog, as appropriate.

The modified nucleotides and the modified nucleotide analogs are not limited to those mentioned herein. Many modified nucleotides and modified nucleotide analogs are known in the art; for example, the teachings of the specification of U.S. Patent No. 8,299,039 to Tachas et al., particularly the teachings in col. 17 to 22, may be applied to the embodiments of the present invention.

A person skilled in the art can select, from these modified nucleotides and nucleotide analogs, the modified nucleotide or nucleotide analog to be used, as appropriate, in consideration of the antisense effect, the affinity for a partial sequence of a transcript of a target gene, and the nuclease resistance. In a certain embodiment, an LNA represented by the following formula (1) may be used: 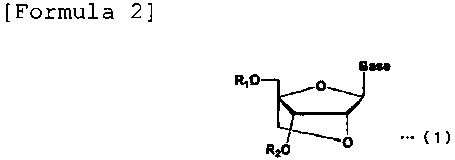 wherein Base represents an aromatic heterocyclic group or aromatic hydrocarbon ring group optionally having a substituent, which is, for example, a base moiety (purine base or pyrimidine base) of a natural nucleoside or a base moiety of an unnatural (modified) nucleoside, wherein examples of modifications of the base moiety are as described above; R₁ and R₂ may be the same or different, and each represent a hydrogen atom, a hydroxy-protecting group for nucleic acid synthesis, an alkyl group, an alkenyl group, a cycloalkyl group, an aryl group, an aralkyl group, an acyl group, a sulfonyl group, a silyl group, a phosphoric acid group, a phosphoric acid group protected by a protecting group for nucleic acid synthesis, or -P(R⁴)R⁵; wherein R⁴ and R⁵ may be the same or different, and each represent a hydroxyl group, a hydroxyl group protected by a protecting group for nucleic acid synthesis, a mercapto group, a mercapto group protected by a protecting group for nucleic acid synthesis, an amino group, a C₁₋₅ alkoxy group, a C₁₋₅ alkylthio group, a C₁₋₆ cyanoalkoxy group, or an amino group substituted with a C₁₋₅ alkyl group.

The compound shown in the chemical formula above is a nucleoside; however, the "LNA", and generally a BNA, in a certain embodiment, also includes a form in which the nucleoside has a phosphate group linked thereto (nucleotide). That is, a BNA such as an LNA may be incorporated into the polynucleotide as a nucleotide.

In one embodiment of the present invention, the "wing region that comprises one or more modified nucleotides and/or nucleotide analogs" is positioned at the 5' end and/or the 3' end of the region comprising at least four contiguous deoxyribonucleotides (hereinafter also referred to as the "DNA gap region").

In one embodiment of the present invention, the region comprising one or more modified nucleotides and/or nucleotide analogs located at the 5' end of the DNA gap region (hereinafter also referred to as the "5'-wing region") and the region comprising one or more modified nucleotides and/or nucleotide analogs located at the 3' end of the DNA gap region (hereinafter also referred to as the "3'-wing region"), which are independent from each other, may comprise at least one modified nucleotide and/or nucleotide analog from those mentioned in the documents concerning antisense technologies, and may further comprise a natural nucleotide (a deoxyribonucleotide or a ribonucleotide) other than the modified nucleotides and/or nucleotide analogs. In general, the 5'-wing region and the 3'-wing region may independently be 1 to 10 bases, preferably 1 to 7 or 2 to 5 bases, for example, 2 to 4 bases, in length.

Furthermore, the types, number, or positions of the modified nucleotides and/or nucleotide analogs and the natural nucleotide in the 5'-wing region and the 3'-wing region may affect the antisense effect and the like to be achieved by the nucleic acid complex in a certain embodiment, and thus, preferred embodiments thereof may vary with the sequence or the like. Although such preferred embodiments cannot be unequivocally stated, a person skilled in the art can determine them by referring to the teachings of documents concerning antisense technologies. Furthermore, a modification can be evaluated as described for the region comprising "at least four contiguous deoxyribonucleotides", as follows: the antisense effect of a modified nucleic acid, preferably a modified double-stranded nucleic acid, is measured, and if the measured value is not significantly lower than that of the nucleic acid, preferably the double-stranded nucleic acid, before being modified, then the modification can be evaluated as a preferred one.

As described in Patent Literature 1, the previously attempted antisense technology involving only RNA or LNAs suppresses translation through binding to target mRNA; however, the effect is typically insufficient. On the other hand, in an antisense technology involving DNA only, because a double-stranded structure composed of DNA and RNA is obtained once the DNA binds to a target gene, this structure becomes a target of RNase H that cleaves the mRNA, such that a potent effect of suppressing the expression of the target gene can be expected. However, because the binding *per se* of the DNA to the target gene is weak, the actual effect is still insufficient. Thus, in the polynucleotide of the active moiety, DNA that is at least four or more bases in length is located in the center, and LNAs (or other BNAs) having a strong binding ability for RNA (i.e., a target transcript) are located at both ends, such that the resulting composite strand promotes cleavage of the target RNA by RNase H.

It is therefore preferred that each of the 5'-wing region and the 3'-wing region comprise one or more modified nucleotides and/or nucleotide analogs. In one embodiment of the present invention, each of the 5'-wing region and the 3'-wing region consists of one or more modified nucleotides and/or nucleotide analogs. Each of the 5'-wing region and the 3'-wing region may comprise a BNA, for example, an LNA.

In one embodiment of the present invention, the polynucleotide of the active moiety consists of one or more deoxyribonucleotides.

In another embodiment of the present invention, the polynucleotide of the active moiety consists of one or more deoxyribonucleotides as well as one or more modified nucleotides and/or nucleotide analogs.

In a further embodiment of the present invention, the polynucleotide of the active moiety consists of at least four contiguous deoxyribonucleotides, as well as a 5'-wing region positioned 5' to the deoxyribonucleotides and a 3'-wing region positioned 3' to the deoxyribonucleotides. In this embodiment, a portion or all of the deoxyribonucleotides may be modified.

In one embodiment of the present invention, the polynucleotide of the active moiety is free of a ribonucleotide.

As described above, the present invention relates to a nucleic acid complex comprising:
(i) an active moiety comprising a polynucleotide comprising at least one or more deoxyribonucleotides and optionally one or more modified nucleotides and/or nucleotide analogs as structural units; and
(ii) a carrier moiety comprising a polynucleotide comprising one or more nucleotides and optionally one or more modified nucleotides and/or nucleotide analogs as structural units, the polynucleotide being at least partially complementary to the polynucleotide of (i) the active moiety, wherein
the polynucleotide of (ii) the carrier moiety comprises at least one bulge.

In one embodiment of the present invention, a portion or all of the deoxyribonucleotides of the polynucleotide of (i) the active moiety are modified.

In a further embodiment of the present invention, the polynucleotide of (i) the active moiety comprises one or more modified nucleotides and/or nucleotide analogs.

In one embodiment of the present invention, the polynucleotide of (i) the active moiety comprises one or more nucleotide analogs, wherein at least one of the nucleotide analogs is optionally modified.

In a further embodiment of the present invention, the polynucleotide of (i) the active moiety comprises at least four contiguous deoxyribonucleotides, and
the polynucleotide comprises:
(a) a 5'-wing region that is positioned 5' to the at least four contiguous deoxyribonucleotides, and comprises one or more modified nucleotides and/or nucleotide analogs; and/or
(b) a 3'-wing region that is positioned 3' to the at least four contiguous deoxyribonucleotides, and comprises one or more modified nucleotides and/or nucleotide analogs.

In this case, the polynucleotide of (i) the active moiety may comprise (a) the 5'-wing region and (b) the 3'-wing region.

In one embodiment of the present invention, the 5'-wing region comprises two to five modified nucleotides and/or nucleotide analogs, and the 3'-wing region comprises two to five modified nucleotides and/or nucleotide analogs. In an alternative embodiment of the present invention, the 5'-wing region comprises one modified nucleotide and/or nucleotide analog, and the 3'-wing region comprises one modified nucleotide and/or nucleotide analog. In this case, each of the 5'-wing region and the 3'-wing region may comprise a sugar-modified nucleotide (i.e., a nucleotide having a modified sugar subunit) and/or a bridged nucleotide as a nucleotide analog. The bridged nucleotide may also be selected from the group consisting of LNA, cEt-BNA, amide BNA (AmNA), and cMOE-BNA. The bridged nucleotide is optionally phosphorothioated. The at least four contiguous deoxyribonucleotides are optionally phosphorothioated.

For example, the polynucleotide of (i) the active moiety consists of a 5'-wing region consisting of one or more phosphorothioated bridged nucleotides, at least four contiguous phosphorothioated deoxyribonucleotides, and a 3'-wing region consisting of one or more phosphorothioated bridged nucleotides. When each of the two wing regions consists of two to five phosphorothioated bridged nucleotides, the polynucleotide can be represented by the following formula (I): 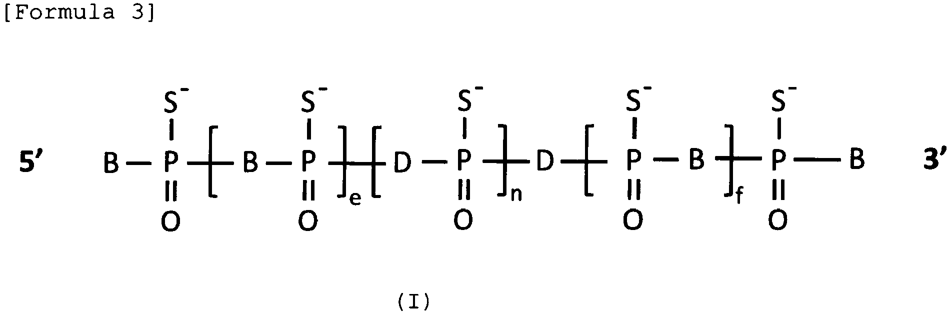 wherein each B independently represents a bridged nucleoside; each D independently represents a deoxyribonucleoside; e represents an integer from 1 to 4; f represents an integer from 1 to 4; and n represents an integer from 3 or more, preferably 3 to 19, more preferably 4 to 15, and still more preferably 5 to 13, for example, 6 to 12.

In formula (I) above, the following moiety (I-1) corresponds to the 5'-wing region: 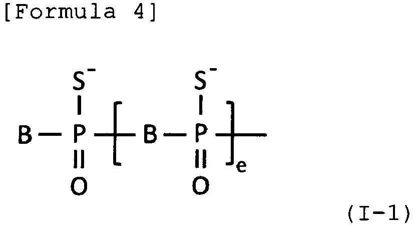 ; the following moiety (I-2) corresponds to the at least four contiguous phosphorothioated deoxyribonucleotides: 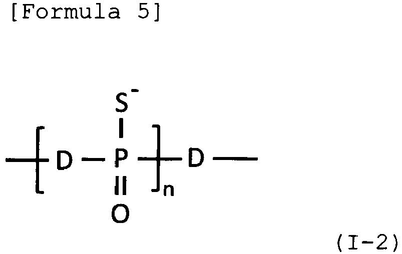 ; and the following moiety (I-3) corresponds to the 3'-wing region:

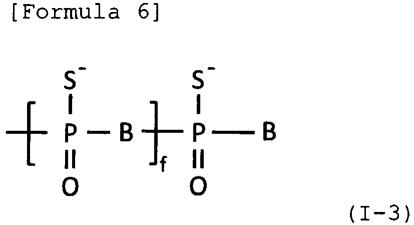

Likewise, in formula (II) shown below, the 5'-wing region, the at least four contiguous deoxyribonucleotides, and the 3'-wing region are represented by the respective corresponding moieties.

In a further embodiment of the present invention, the 5'-wing region consists of one to five sugar-modified ribonucleotides, and the 3'-wing region consists of one to five sugar-modified ribonucleotides, wherein the sugar-modified ribonucleotides are optionally phosphorothioated. For example, the polynucleotide of (i) the active moiety consists of a 5'-wing region consisting of one or more phosphorothioated sugar-modified ribonucleotides, at least four contiguous phosphorothioated deoxyribonucleotides, and a 3'-wing region consisting of one or more phosphorothioated sugar-modified ribonucleotides, and can be represented by the following formula (II): 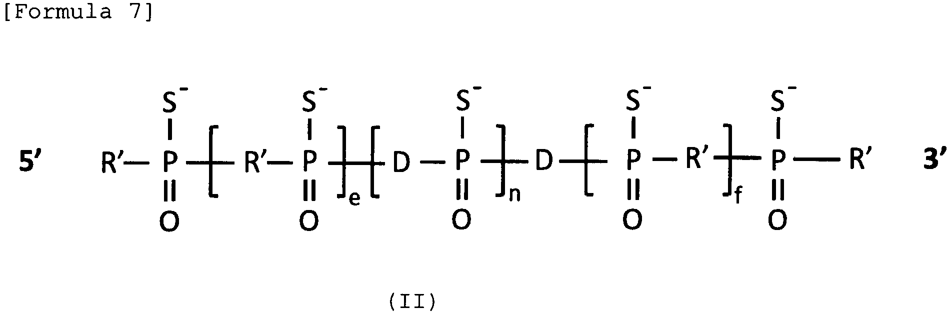 wherein each R' independently represents a sugar-modified ribonucleoside; each D independently represents a deoxyribonucleoside; e represents an integer from 0 to 4; f represents an integer from 0 to 4; and n represents an integer from 3 or more, preferably 3 to 19, more preferably 4 to 15, and still more preferably 5 to 13, for example, 6 to 12.

In this case, the sugar modification may be selected from the group consisting of 2'-O-methylation, 2'-O-methoxyethylation (2'-MOE modification), 2'-O-aminopropylation (2'-AP modification), and 2'-fluorination.

The number of nucleotides forming the 5'-wing region and the number of nucleotides forming the 3'-wing region may be the same or different. Thus, in each of formulas (I) and (II) above, e and f may be the same or different. For example, in formula (I) and/or formula (II) above, the following relation may hold: e = 0 and f = 0 (in formula (II)); e = 1 and f = 1; e = 2 and f = 2; or e = 3 and f = 3; alternatively, e = 1 and f = 2; or e = 2 and f = 1.

In another embodiment of the present invention, the polynucleotide of (i) the active moiety consists of at least four contiguous phosphorothioated deoxyribonucleotides. In this embodiment, the polynucleotide can be represented by the following formula (III): 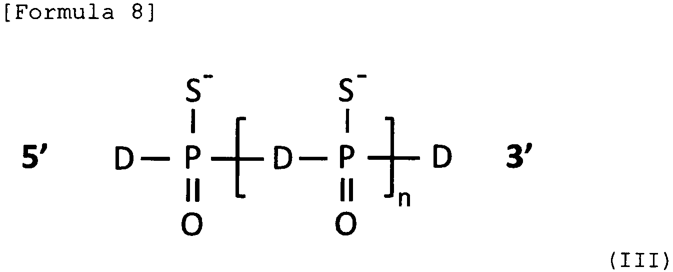 wherein each D independently represents a deoxyribonucleoside; and n represents an integer from 2 or more, preferably 2 to 18, more preferably 3 to 14, and still more preferably 4 to 12, for example, 5 to 11.

In a further embodiment of the present invention, the polynucleotide of (i) the active moiety consists of at least four contiguous unmodified deoxyribonucleotides. In this embodiment, the polynucleotide can be represented by the following formula (IV): 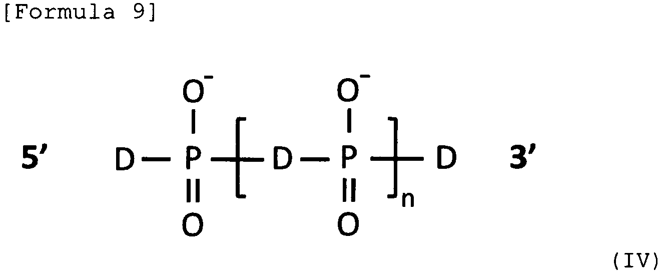 wherein each D independently represents a deoxyribonucleoside; and n represents an integer from 2 or more, preferably 2 to 18, more preferably 3 to 14, and still more preferably 4 to 12, for example, 5 to 11.

In one preferred embodiment of the present invention, in the active moiety, the 5'-wing region is positioned at the 5' end of the polynucleotide, i.e., comprises structural units positioned at the 5' end of the polynucleotide. In a further preferred embodiment of the present invention, in the active moiety, the 3'-wing region is positioned at the 3' end of the polynucleotide, i.e., comprises structural units positioned at the 3' end of the polynucleotide.

As described above, in the present invention, the polynucleotide of the carrier moiety is a polynucleotide that is at least partially complementary to the polynucleotide of the active moiety. The base sequence of the polynucleotide of the carrier moiety and the base sequence of the polynucleotide of the active moiety need not be fully complementary to each other, so long as the two polynucleotides can at least partially form a duplex, and may have, for example, at least 70% or more, preferably 80% or more, and more preferably 90% or more (for example, 95%, 96%, 97%, 98%, or 99% or more) complementarity. The complementarity is calculated as the complementarity in the entire duplex-forming region, as described above; however, because the polynucleotide of the carrier moiety comprises a bulge structure, the bulge structure is excluded from the duplex-forming region, and then the complementarity is calculated. That is, when the complementarity is calculated, the bulge structure is not included in the duplex-forming region.

In one preferred embodiment of the present invention, the two polynucleotides have 100% complementarity. In this case, the complementarity is calculated as the complementarity in the duplex-forming region (entire duplex-forming region) from which the bulge structure is excluded as described above. In this embodiment, when, for example, the length of the polynucleotide of the carrier moiety (excluding the bulge-forming portion) is longer than that of the polynucleotide of the active moiety, the entire polynucleotide of the active moiety is fully complementary to a portion of the polynucleotide of the carrier moiety (excluding the bulge-forming portion), and the fully complementary portion defines the duplex-forming region. When the length of the polynucleotide of the carrier moiety (excluding the bulge-forming portion) is shorter than that of the polynucleotide of the active moiety, the entire polynucleotide of the carrier moiety (excluding the bulge-forming portion) is fully complementary to a portion of the polynucleotide of the active moiety, and the fully complementary portion defines the duplex-forming region. When the polynucleotide of the active moiety and the polynucleotide of the carrier moiety (excluding the bulge-forming portion) have the same length, the two polynucleotides are fully complementary to each other across the entire length, excluding the bulge structure.

The polynucleotide of the carrier moiety comprises one or more nucleotides and optionally one or more modified nucleotides and/or nucleotide analogs, as described above. The polynucleotide of the carrier moiety further comprises at least one bulge. The polynucleotide herein may comprise one or more deoxyribonucleotides and/or ribonucleotides as the nucleotides. In one embodiment of the present invention, the polynucleotide may comprise one or more deoxyribonucleotides and one or more ribonucleotides as the nucleotides.

From the viewpoint of having high resistance to a nuclease such as an RNase, the nucleotides of the polynucleotide of the carrier moiety (deoxyribonucleotides and/or ribonucleotides) may also have modifications as described above for the polynucleotide of the active moiety.

The polynucleotide of the carrier moiety may comprise one or more modified nucleotides and/or nucleotide analogs as described for the polynucleotide of the active moiety, from the viewpoint of allowing the antisense effect to be readily demonstrated by suppressing the degradation of the polynucleotide of the carrier moiety by an RNase such as RNase A until delivery into the nucleus of a specific cell, while allowing the polynucleotide to be degraded by RNase H in the specific cell.

As in the polynucleotide of the active moiety, when the polynucleotide of the carrier moiety comprises one or more nucleotide analogs, the nucleotide analogs are optionally modified.

Since the number or positions of such modifications may affect the antisense effect and the like to be achieved by the nucleic acid complex in a certain embodiment, there are preferred embodiments for the number and the positions of modifications of the nucleotide analogs contained in the polynucleotide of the carrier moiety. These preferred embodiments cannot be unequivocally stated since they may vary with the type, sequence, and the like of the nucleic acid to be modified; however, the preferred embodiments can be specified by measuring the antisense effect of a modified nucleic acid complex, as described for the polynucleotide of the active moiety.

In one embodiment of the present invention, the polynucleotide of the carrier moiety has one or more modified deoxyribonucleotides and/or nucleotide analogs in the region complementary to the 5'-wing region and/or the 3'-wing region of the polynucleotide of the active moiety. In this case, the modified deoxyribonucleotides and/or the nucleotide analogs are expected to have the effect of suppressing degradation by an enzyme such as an RNase. In one further embodiment of the present invention, in the polynucleotide of the carrier moiety, all of the region complementary to the region of the polynucleotide of the active moiety comprising a nucleotide analog is modified, and a portion of the region complementary to the region of the polynucleotide of the active moiety comprising a modified nucleic acid is modified. In this case, so long as the modified region of the polynucleotide of the carrier moiety comprises the above-described portion, it may be longer than, shorter than, or the same in length as the region of the polynucleotide of the active moiety comprising a modified nucleic acid.

The polynucleotide of the carrier moiety is not particularly limited in length, and is at least 8 bases, at least 10 bases, at least 12 bases, at least 13 bases, at least 14 bases, at least 15 bases, or at least 16 bases in length. The length may be preferably 100 bases or less, 45 bases or less, 35 bases or less, 30 bases or less, 25 bases or less, 24 bases or less, 23 bases or less, 22 bases or less, 21 bases or less, or 20 bases or less. The range of lengths is preferably from 10 to 35 bases, more preferably from 12 to 25 bases, and still more preferably from 13 to 22 bases. In general, the length is selected according to the effect upon the delivery to a target site, as well as other factors such as the size or number of bulges, cost, and synthesis yield.

In one embodiment of the present invention, the polynucleotide of the active moiety and the polynucleotide of the carrier moiety may be the same or different in length when compared with the bulge being excluded from the polynucleotide of the carrier moiety.

In one embodiment of the present invention, the polynucleotide of the active moiety is greater in length than the polynucleotide of the carrier moiety when compared with the bulge being excluded from the polynucleotide of the carrier moiety.

In another embodiment of the present invention, the polynucleotide of the active moiety is smaller in length than the polynucleotide of the carrier moiety when compared with the bulge being excluded from the polynucleotide of the carrier moiety.

As described above, the polynucleotide of the carrier moiety comprises at least one bulge. As used herein, the "bulge" refers to a bulged structure formed in a double-stranded region between the polynucleotide of the active moiety and the polynucleotide of the carrier moiety formed by annealing (also simply referred to as the "duplex-forming region", hereinafter), wherein the bulged structure is formed of nucleotide(s) (and/or modified nucleotide(s)/nucleotide analog(s)) in the latter polynucleotide that are flipped out of the double-stranded nucleic acid without forming base pair(s), because one base, or two or more contiguous bases corresponding to the latter polynucleotide are missing in the former polynucleotide.

The number of bulges is not particularly limited so long as it is within the range that allows the formation of a duplex. In one embodiment of the present invention, the polynucleotide of the carrier moiety may comprise one to four bulges, for example, one to three, or two or three, bulges. In one further embodiment of the present invention, the polynucleotide of the carrier moiety comprises one, two, three, or four bulges.

The position of a bulge is not particularly limited so long as a bulge structure can be formed at the position. For example, when each of the polynucleotide of the active moiety and the polynucleotide of the carrier moiety has a length of 10 to 35 bases, a bulge may be introduced at a corresponding position of the polynucleotide of the carrier moiety such that it is, for example, at any of positions 2 to 15, for example, positions 6 to 11 (for example, position 6, 7, 8, 9, 10, or 11), from the 5' end of the duplex-forming region (5' end of the polynucleotide of the active moiety in the duplex-forming region). When the polynucleotide of the carrier moiety has a plurality of bulges, a first bulge may be introduced at a corresponding position of the polynucleotide of the carrier moiety such that it is, for example, at any of positions 2 to 15, for example, positions 6 to 11, from the 5' end of the duplex-forming region (5' end of the polynucleotide of the active moiety in the duplex-forming region), and other bulges may be introduced at every one to several positions, for example, every one to three, or one or two positions, from the first bulge.

The number of nucleotides or the like (the number of nucleotides that do not base pair with the polynucleotide of the active moiety) that form a bulge is not particularly limited so long as a bulge structure can be formed. In one embodiment of the present invention, one bulge may be composed of one to seven, for example, one to five, or two to four (three, for example) nucleotides or the like.

When the polynucleotide of the carrier moiety has a plurality of bulges, the bulges may be composed of the same number of nucleotides or the like. Alternatively, each of the bulges may be independently composed of a different number of nucleotides or the like.

In one embodiment of the present invention, the bulge is composed of one or more nucleotides, modified nucleotides, and/or nucleotide analogs.

In one further embodiment of the present invention, one or more deoxyribonucleotides and/or ribonucleotides (both may be modified or unmodified) are used as the nucleotides forming the bulge.

A person skilled in the art can select the structural units of the polynucleotide of the carrier moiety, as appropriate, such that the bulge is formed, in accordance with the types or base sequence of the polynucleotide of the active moiety.

In one preferred embodiment of the present invention, a portion or all of the nucleotides forming the bulge are ribonucleotides, preferably uracil (U).

In the nucleic acid complex of the present invention (preferably the double-stranded nucleic acid complex) comprising at least one bulge in the polynucleotide of the carrier moiety, the nucleic acid complex is moderately attacked by a DNase or RNase at the bulge structure (i.e., the portion where the duplex is not formed), such that only the carrier moiety is moderately cleaved (in any of the cases where the polynucleotide of the carrier moiety comprises one or more deoxyribonucleotides, comprises one or more ribonucleotides, and comprises a combination thereof) before delivery to target mRNA in cells or *in vivo,* leaving only the polynucleotide of the active moiety. It is believed that this promotes favorable formation of a duplex between the polynucleotide of the active moiety and the target mRNA. In this case, the duplex between the polynucleotide of the active moiety and the target mRNA is recognized by RNase H to allow efficient cleavage of the target mRNA. It is believed that this achieves a more potent effect of suppressing the expression of the target mRNA.

In one embodiment of the present invention, the polynucleotide of the carrier moiety comprises at least one mismatch compared to the polynucleotide of the active moiety.

The "mismatch" refers to the situation where, at a certain position in the duplex-forming region between the polynucleotide of the active moiety and the polynucleotide of the carrier moiety formed by annealing (also simply referred to as the "duplex-forming region", hereinafter), a Watson-Crick base pair is not formed between the nucleotides in the two polynucleotides.

The mismatch as used herein, however, is intended to mean that a base pair is not formed only at the above-described position, even though the polynucleotide of the active moiety and the polynucleotide of the carrier moiety have the same number of nucleotides and/or nucleotide analogs (also collectively referred to as "nucleotides or the like", hereinafter) in the duplex-forming region, and base pairs are formed at all positions excluding that position. Thus, the "mismatch" does not include a structure such as an overhang or a bulge resulting from a difference in length between the two polynucleotides in the duplex-forming region. This is intended to distinguish a mismatch from an overhang or bulge, and does not preclude a mismatch and a bulge (and/or an overhang) from being present simultaneously in the nucleic acid complex, preferably the double-stranded nucleic acid complex, of the present invention.

Thus, the phrase "the polynucleotide of the carrier moiety comprises at least one mismatch compared to the polynucleotide of the active moiety" is intended to mean that the polynucleotide of the carrier moiety has the same length as that of the polynucleotide of the active moiety in the duplex-forming region excluding the bulge structure, and includes at least one nucleotide or the like that does not form a Watson-Crick base pair between the two polynucleotides in the duplex-forming region. Examples of mismatches include A-G, C-A, U-C, A-A, G-G, and C-C, or U-G, U-C, and U-T in the case of using ribonucleotides. Likewise, examples of mismatches also include I-G, non-base residue-nucleotide or the like, and non-cyclic residue-nucleotide or the like. In a broad sense, the mismatch as used herein also includes any conversion at a specific position that reduces the thermodynamic stability at or near the position such that the thermodynamic stability of the duplex at the position becomes lower than that of a Watson-Crick base pair at the position.

In the present invention, the polynucleotide of the carrier moiety in the nucleic acid complex may comprise at least one mismatch compared to the polynucleotide of the active moiety. Likewise, the polynucleotide of the active moiety may comprise at least one mismatch compared to the polynucleotide of the carrier moiety and/or a target transcript (specifically, the region that forms a duplex with the polynucleotide of the active moiety). In particular, the polynucleotide of active moiety may comprise at least one mismatch compared to a target transcript. The number of mismatches is not particularly limited so long as it is within the range that does not hinder the formation of a double-stranded nucleic acid. When a plurality of mismatches are included, the mismatches may be separate from one another, may be contiguously present, or may be present in a combined manner thereof. In one embodiment of the present invention, the polynucleotide of the carrier moiety may comprise one to seven mismatches, for example, one to five, one to four, or two or three mismatches, compared to the polynucleotide of the active moiety. In one further embodiment of the present invention, the polynucleotide of the carrier moiety comprises one, two, three, four, five, six, or seven mismatches compared to the polynucleotide of the active moiety. Likewise, the polynucleotide of the active moiety may comprise one to seven mismatches, for example, one to five, one to four, or two or three mismatches, compared to the polynucleotide of the carrier moiety and/or a target transcript (specifically, the region that forms a duplex with the polynucleotide of the active moiety). In one further embodiment of the present invention, the polynucleotide of the active moiety comprises one, two, three, four, five, six, or seven mismatches compared to the polynucleotide of the carrier moiety and/or a target transcript (specifically, the region that forms a duplex with the polynucleotide of the active moiety). The polynucleotide of the carrier moiety may comprise at least one mismatch compared to the polynucleotide of the active moiety, and simultaneously, the polynucleotide of the active moiety may also comprise at least one mismatch compared to the polynucleotide of the carrier moiety and/or a target transcript (specifically, the region that forms a duplex with the polynucleotide of the active moiety).

In one embodiment of the present invention, the polynucleotide of the carrier moiety is free of a mismatch compared to the polynucleotide of the active moiety. In a further embodiment of the present invention, the polynucleotide of the active moiety is free of a mismatch compared to the polynucleotide of the carrier moiety and/or a target transcript, and particularly, is free of a mismatch compared to the target transcript.

The position of a mismatch is not particularly limited so long as a mismatch structure can be formed at the position without hindering the formation of a bulge.

For example, when each of the polynucleotide of the active moiety and the polynucleotide of the carrier moiety has a length of 10 to 35 bases, a mismatch may be introduced, for example, at any of positions 2 to 15, for example, positions 6 to 11 (for example, position 6, 7, 8, 9, 10, or 11), from the 5' end of the duplex-forming region (5' end of the polynucleotide of the active moiety in the duplex-forming region). When the polynucleotide of the carrier moiety and/or the polynucleotide of the active moiety have a plurality of mismatches independently or in total, a first mismatch may be introduced, for example, at any of positions 2 to 15, for example, positions 6 to 11 (for example, position 6, 7, 8, 9, 10, or 11), from the 5' end of the duplex-forming region (5' end of the polynucleotide of the active moiety in the duplex-forming region), and other mismatches may be introduced at every one or two positions from the first mismatch, contiguously with the first mismatch, or in a combined manner thereof.

For example, when the nucleic acid complex of the present invention has at least one mismatch, the nucleic acid complex is moderately attacked by a DNase or RNase at the mismatch, i.e., the portion where the duplex is not formed, such that only the carrier moiety is moderately cleaved (in any of the cases where the polynucleotide of the carrier moiety comprises one or more deoxyribonucleotides, comprises one or more ribonucleotides, comprises one or more modified nucleotides, and comprises a combination thereof) before delivery to target mRNA in cells or *in vivo,* leaving only the polynucleotide of the active moiety. It is believed that this promotes favorable formation of a duplex between the polynucleotide of the active moiety and the target mRNA. In this case, the duplex between the polynucleotide of the active moiety and the target mRNA is recognized by RNase H to allow efficient cleavage of the target mRNA. It is believed that this achieves a more potent effect of suppressing the expression of the target mRNA.

When the nucleic acid complex of the present invention has a mismatch as described above, the polynucleotide of the active moiety and the polynucleotide of the carrier moiety (excluding the bulge) are not fully complementary to each other; however, as described above, the two polynucleotides may not be fully complementary to each other in the present invention. In one preferred embodiment of the present invention, when the polynucleotide of the carrier moiety comprises a mismatch, the two polynucleotides have 100% complementarity in the duplex-forming region excluding the mismatch and the bulge. This also applies to the case where the polynucleotide of the active moiety has at least one mismatch compared to a target transcript.

In one embodiment of the present invention, the polynucleotide of the carrier moiety comprises at least one bulge and at least one mismatch.

In an alternative embodiment of the present invention, the polynucleotide of the carrier moiety is free of a mismatch.

For example, from the viewpoint of allowing the polynucleotide of the carrier moiety to be moderately cleaved, the polynucleotide of the carrier moiety may include at least one ribonucleotide as described above, instead of or in addition to having a mismatch as described above. Because a ribonucleotide is typically less resistant to a nuclease than a deoxyribonucleotide (including a modified deoxyribonucleotide) or a nucleotide analog, the inclusion of such a ribonucleotide is believed to promote moderate cleavage of the polynucleotide of the carrier moiety starting from the site at which the ribonucleotide has been introduced.

Thus, in one embodiment of the present invention, the polynucleotide of the carrier moiety comprises one or more ribonucleotides as structural units.

In a further embodiment of the present invention, the polynucleotide of the carrier moiety comprises at least one mismatch compared to the polynucleotide of the active moiety, and comprises one or more ribonucleotides as structural units.

In one further embodiment of the present invention, the polynucleotide of the carrier moiety comprises at least one mismatch compared to the polynucleotide of the active moiety, and comprises one or more ribonucleotides as structural units, wherein at least one, for example, one, two, three, or four, of the ribonucleotides form the mismatches.

The number of the ribonucleotides to be included in the polynucleotide of the carrier moiety is not particularly limited. The polynucleotide of the carrier moiety may comprise, for example, 1 to 90%, preferably 5 to 70% or 10 to 50%, of ribonucleotides, relative to a total number of nucleotides in the polynucleotide. In one embodiment of the present invention, the polynucleotide of the carrier moiety may comprise, for example, 10, 20, 30, 40, 50, 60, 70, 80, or 90% of ribonucleotides relative to a total number of nucleotides in the polynucleotide. In an alternative embodiment of the present invention, the polynucleotide of the carrier moiety consists of one or more ribonucleotides only.

In a further embodiment of the present invention, the polynucleotide of the carrier moiety may comprise one to twelve ribonucleotides, for example, two to ten, three to eight, or four to seven ribonucleotides. In one further embodiment of the present invention, the polynucleotide of the carrier moiety comprises one, two, three, four, five, six, seven, eight, nine, ten, eleven, twelve, thirteen, fourteen, fifteen, sixteen, seventeen, eighteen, nineteen or twenty ribonucleotides.

When a plurality of ribonucleotides are included, the ribonucleotides may be separate from one another, may be contiguously present, or may be present in a combined manner thereof. In one embodiment of the present invention, the polynucleotide of the carrier moiety comprises a plurality of ribonucleotides, wherein the ribonucleotides alternate or substantially alternate with the deoxyribonucleotides. In this case, one, two, three, four, five, or six ribonucleotides may alternate (or substantially alternate) with one, two, three, four, five, or six deoxyribonucleotides.

The positions of the ribonucleotides in the polynucleotide of the carrier moiety are not particularly limited. When the polynucleotide of the carrier moiety has a length of 10 to 35 bases, it may comprise a ribonucleotide at any of positions 1 to 35, for example, any of positions 2 to 30, 3 to 25, 4 to 20, 5 to 15, and 6 to 11 (for example, position 6, 7, 8, 9, 10, or 11), from the 5' end. When the polynucleotide of the carrier moiety comprises a plurality of ribonucleotides, a first ribonucleotide may be introduced, for example, at any of positions 1 to 15, for example, positions 6 to 11 (for example, position 6, 7, 8, 9, 10, or 11), from the 5' end, and other ribonucleotides may be included at every one or two positions from the first ribonucleotide, contiguously with the first ribonucleotide, or in a combined manner thereof. For example, when the polynucleotide of the carrier moiety comprises a plurality of contiguous ribonucleotides, the polynucleotide can be represented by the following formula (V): 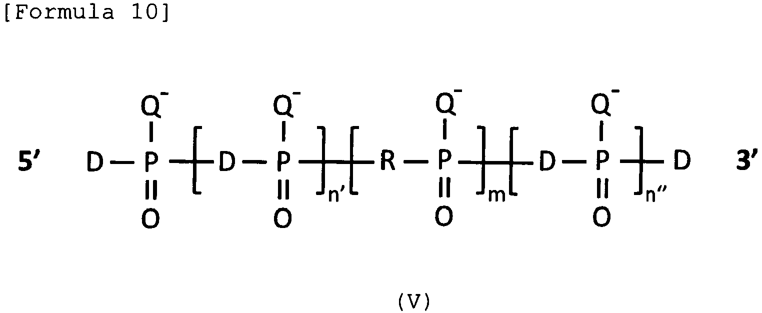 wherein each D independently represents a deoxyribonucleoside; each R independently represents a ribonucleoside; each Q independently represents S or O; n' represents an integer from 0 or more; n'' represents an integer from 1 or more; and m represents an integer from 2 or more; wherein the sum of n' + n" + m is preferably an integer from 8 to 33, more preferably 10 to 23, and still more preferably 11 to 18.

In formula (V) above, the case where n' = 4 to 9 corresponds to the above-described embodiment where the first ribonucleotide is introduced at any of positions 6 to 11. In formula (V) above, m may be an integer from 2 to 6, for example; n' may be an integer from 3 to 15, 4 to 10, or 5 to 8, for example; and n" may be an integer from 3 to 15, 4 to 10, or 5 to 8, for example. The case where m = 1, i.e., where the polynucleotide comprises one ribonucleotide, is also one embodiment of the present invention, as described above.

For example, the polynucleotide represented by SEQ ID NO: 2 in Example 1, which has a length of 17 bases, and comprises one ribonucleotide, wherein the ribonucleotide is included at position 9 from the 5' end, corresponds to formula (V) above, wherein Q = O, n' = 7, m = 1, and n" = 7. Moreover, the polynucleotides represented by SEQ ID NOS: 3 to 5 have a length of 18 to 20 bases, respectively, comprise two to four ribonucleotides, respectively, and each have a first ribonucleotide at position 9 from the 5' end, and comprise the other ribonucleotide(s) contiguously with the first ribonucleotide. Each of these polynucleotides is represented by:
SEQ ID NO: 3: formula (V), wherein Q = O, n' = 7, m = 2, and n" = 7;
SEQ ID NO: 4: formula (V), wherein Q = O, n' = 7, m = 3, and n" = 7;
SEQ ID NO: 5: formula (V), wherein Q = O, n' = 7, m = 4, and n" = 7.

Likewise, when the polynucleotide of the carrier moiety comprises one or more ribonucleotides, wherein at least one of the ribonucleotides produces a mismatch, the polynucleotide may comprise the ribonucleotides at similar positions.

Furthermore, in one embodiment of the present invention, the polynucleotide of the carrier moiety may comprise a ribonucleotide at the bulge, as described above.

If the number of ribonucleotides is large (particularly if the number of contiguous ribonucleotides is large), a DNA/RNA duplex having a certain length is formed between the polynucleotide of the active moiety and the polynucleotide of the carrier moiety, which is then readily recognized by RNase H. As a result, the effect of allowing proper cleavage of the polynucleotide of the carrier moiety before delivery to target mRNA in cells or *in vivo* can also be expected.

To regulate the degree of cleavage, the ribonucleotides may have modifications as described above for the deoxyribonucleotides.

In one embodiment of the present invention, the nucleic acid complex may have an overhang.

As used herein, the "overhang" refers to a non-base-pairing terminal nucleotide (a modified nucleotide and/or a nucleotide analog) resulting from a single-stranded region in a duplex where one strand extends beyond an end of the other strand complementary thereto in the duplex. Each overhang comprises at least one nucleotide (a modified nucleotide and/or a nucleotide analog). Preferably, each overhang is a two-nucleotide overhang. The nucleotides forming the overhang may be selected as desired. The nucleotides of the overhang may or may not base pair with a target transcript. Examples of the two-nucleotide overhang include, although not limited to, UU, TT, AA, GG, CC, AC, CA, AG, GA, GC, and CG.

Overhang(s) may be positioned at the 5' end of the polynucleotide of the active moiety; the 3' end of the polynucleotide of the active moiety; the 5' and 3' ends of the polynucleotide of the active moiety; the 5' end of the polynucleotide of the carrier moiety; the 3' end of the polynucleotide of the carrier moiety; the 5' and 3' ends of the polynucleotide of the carrier moiety; the 5' end of the polynucleotide of the active moiety and the 5' end of the polynucleotide of the carrier moiety; or the 3' end of the polynucleotide of the active moiety and the 3' end of the polynucleotide of the carrier moiety.

In another embodiment of the present invention, the nucleic acid complex is free of an overhang.

In an alternative embodiment of the present invention, the polynucleotide of the carrier moiety may comprise at least four contiguous deoxyribonucleotides, and may comprise a 5'-wing region positioned 5' to the deoxyribonucleotides and/or a 3'-wing region positioned 3' to the deoxyribonucleotides, as described for the polynucleotide of the active moiety.

In one embodiment of the present invention, the polynucleotide of the carrier moiety consists of one or more deoxyribonucleotides only, or the polynucleotide of the carrier moiety excluding the bulge consists of one or more deoxyribonucleotides only. In this embodiment as well, the nucleic acid complex can favorably suppress the level of a target transcript. This is believed to occur by means of the following mechanism: before delivery to the target transcript, the DNA-DNA duplex formed between the polynucleotide of the active moiety and the polynucleotide of the carrier moiety is recognized by a DNase that degrades the polynucleotide of the carrier moiety. The remaining polynucleotide of the active moiety subsequently forms a DNA-RNA duplex with the target transcript, and then this structure is recognized and degraded by RNase H.

In another embodiment of the present invention, the polynucleotide of the carrier moiety consists of one or more deoxyribonucleotides and one or more ribonucleotides only. In an alternative embodiment of the present invention, the polynucleotide of the carrier moiety consists of one or more deoxyribonucleotides as well as one or more modified nucleotides and/or nucleotide analogs only, consists of one or more ribonucleotides as well as one or more modified nucleotides and/or nucleotide analogs only, or consists of one or more deoxyribonucleotides, one or more ribonucleotides, as well as one or more modified nucleotides and/or nucleotide analogs only.

In a further embodiment of the present invention, the polynucleotide of the carrier moiety consists of at least four contiguous deoxyribonucleotides, as well as a 5'-wing region positioned 5' to the deoxyribonucleotides and a 3'-wing region positioned 3' to the deoxyribonucleotides.

In a further embodiment of the present invention, the polynucleotide of the carrier moiety consists of at least four contiguous deoxyribonucleotides; a 5'-wing region positioned 5' to the deoxyribonucleotides and a 3'-wing region positioned 3' to the deoxyribonucleotides; and one or more ribonucleotides, wherein the ribonucleotides may be present in the 5'-wing region and/or the 3'-wing region. In this case, some of the at least four contiguous deoxyribonucleotides may be substituted with ribonucleotides.

In one embodiment of the present invention, the polynucleotide of the carrier moiety is free of a ribonucleotide.

In an alternative embodiment of the present invention, the deoxyribonucleotides of the polynucleotide of the carrier moiety are unmodified.

In a further alternative embodiment of the present invention, the ribonucleotides of the polynucleotide of the carrier moiety are unmodified.

As described above, the nucleic acid complex of the present invention comprises:
(ii) a carrier moiety comprising a polynucleotide comprising one or more nucleotides and optionally one or more modified nucleotides and/or nucleotide analogs as structural units, the polynucleotide being at least partially complementary to the polynucleotide of (i) the active moiety, wherein
the polynucleotide of (ii) the carrier moiety comprises at least one bulge. In one embodiment of the present invention, the polynucleotide of (ii) the carrier moiety may further comprise one or more deoxyribonucleotides as structural units. In this case, the polynucleotide of (ii) the carrier moiety may consist of one or more deoxyribonucleotides. Furthermore, the polynucleotide of (ii) the carrier moiety may comprise one or more ribonucleotides as structural units. In one embodiment of the present invention, the polynucleotide of (ii) the carrier moiety consists of one or more ribonucleotides and one or more deoxyribonucleotides. In these embodiments, a portion or all of the ribonucleotides may be modified, or the ribonucleotides may be unmodified. Likewise, a portion or all of the deoxyribonucleotides may be modified, or the deoxyribonucleotides may be unmodified. For example, the modified deoxyribonucleotides may be base-modified nucleotides (i.e., nucleotides having modified base subunits), and preferably abasic nucleotides (which may correspond to the "non-base residues" described above). In one embodiment, the modified deoxyribonucleotides have no modifications other than the base modification.

In one embodiment of the present invention, the bulge is formed of one or more deoxyribonucleotides and/or ribonucleotides.

For example, the bulge is formed of one or more deoxyribonucleotides. In this case, a portion or all of the deoxyribonucleotides may be modified, or the deoxyribonucleotides may be unmodified. In one preferred embodiment of the present invention, the bulge is composed of one or more modified deoxyribonucleotides, preferably one or more base-modified deoxyribonucleotides, and particularly one or more abasic deoxyribonucleotides. For example, the polynucleotide of the carrier moiety consists of one or more unmodified deoxyribonucleotides and one or more modified deoxyribonucleotides, wherein the modified deoxyribonucleotides (preferably at least two, for example, three, four, five, or six contiguous modified deoxyribonucleotides) form the bulge. In this case, the modified deoxyribonucleotides are preferably base-modified deoxyribonucleotides, and more preferably abasic deoxyribonucleotides. Particularly preferably, the modified deoxyribonucleotides have no modifications other than the base modification.

In an alternative preferred embodiment of the present invention, the bulge is composed of one or more unmodified deoxyribonucleotides (for example, deoxyinosine or thymidine), preferably at least two, for example, three, four, five, or six contiguous unmodified deoxyribonucleotides having the same base sequence (for example, deoxyinosine or thymidine).

Alternatively, the bulge may be formed of one or more ribonucleotides. In this case, a portion or all of the ribonucleotides may be modified, or the ribonucleotides may be unmodified. In one preferred embodiment of the present invention, the bulge is composed of one or more unmodified ribonucleotides. For example, the polynucleotide of the carrier moiety consists of one or more unmodified deoxyribonucleotides and one or more unmodified ribonucleotides, wherein the unmodified ribonucleotides (for example, inosine or uracil), preferably at least two, for example, three, four, five, or six contiguous unmodified ribonucleotides having the same base sequence (for example, deoxyinosine or thymidine), form the bulge.

In one preferred embodiment of the present invention, the present invention relates to a double-stranded nucleic acid complex comprising:
(i) an active moiety comprising a polynucleotide comprising, as structural units, at least four contiguous phosphorothioated deoxyribonucleotides, a 5'-wing region that is positioned 5' to the at least four contiguous deoxyribonucleotides, and consists of two to five phosphorothioated bridged nucleotides, and a 3'-wing region that is positioned 3' to the at least four contiguous deoxyribonucleotides, and consists of two to five phosphorothioated bridged nucleotides; and
(ii) a carrier moiety comprising a polynucleotide consisting of one or more unmodified deoxyribonucleotides as structural units, or consisting of one or more unmodified ribonucleotides and one or more unmodified deoxyribonucleotides as structural units, the polynucleotide being at least partially complementary to the polynucleotide of (i) the active moiety, wherein
the polynucleotide of (ii) the carrier moiety comprises at least one bulge.

In another preferred embodiment of the present invention, the present invention relates to a double-stranded nucleic acid complex comprising:
(i) an active moiety comprising a polynucleotide comprising, as structural units, at least four contiguous phosphorothioated deoxyribonucleotides, a 5'-wing region that is positioned 5' to the at least four contiguous deoxyribonucleotides, and consists of two to five phosphorothioated bridged nucleotides, and a 3'-wing region that is positioned 3' to the at least four contiguous deoxyribonucleotides, and consists of two to five phosphorothioated bridged nucleotides; and
(ii) a carrier moiety comprising a polynucleotide consisting of one or more unmodified ribonucleotides and one or more unmodified deoxyribonucleotides as structural units, the polynucleotide being at least partially complementary to the polynucleotide of (i) the active moiety, wherein
the polynucleotide of (ii) the carrier moiety comprises at least one bulge, and the bulge is formed of one or more unmodified ribonucleotides.

Furthermore, the present invention relates to a double-stranded nucleic acid complex comprising:
(i) an active moiety comprising a polynucleotide comprising, as structural units, at least four contiguous phosphorothioated deoxyribonucleotides, a 5'-wing region that is positioned 5' to the at least four contiguous deoxyribonucleotides, and consists of two to five phosphorothioated bridged nucleotides, and a 3'-wing region that is positioned 3' to the at least four contiguous deoxyribonucleotides, and consists of two to five phosphorothioated bridged nucleotides; and
(ii) a carrier moiety comprising a polynucleotide consisting of one or more unmodified ribonucleotides and one or more unmodified deoxyribonucleotides as structural units, the polynucleotide being at least partially complementary to the polynucleotide of (i) the active moiety, wherein
the polynucleotide of (ii) the carrier moiety comprises at least one bulge, and the bulge is formed of one or more unmodified ribonucleotides, and
the polynucleotide of (i) the active moiety comprises no mismatch compared to the polynucleotide of (ii) the carrier moiety and/or a target transcript.

In one preferred embodiment of the present invention, in the polypeptide of the carrier moiety, the 5'-wing region is positioned at the 5' end of the polynucleotide, i.e., comprises structural units positioned at the 5' end of the polynucleotide. In a further preferred embodiment of the present invention, in the carrier moiety, the 3'-wing region is positioned at the 3' end of the polynucleotide, i.e., comprises structural units positioned at the 3' end of the polynucleotide.

In the present invention, various embodiments of nucleic acid complexes, preferably double-stranded complexes, can be prepared by combining, as appropriate, the embodiments concerning the polynucleotide of the active moiety and the embodiments concerning the polynucleotide of the carrier moiety described above.

In one embodiment of the present invention, the nucleic acid complex is a nucleic acid complex for reducing the expression of a target transcript, for example, a target gene, in a cell or mammal.

In one further embodiment of the present invention, the polynucleotide of the active moiety is an antisense strand complementary to any region of mRNA of a target gene. In one embodiment of the present invention, the target gene is human bcl-2 or human STAT3. In an alternative embodiment of the present invention, the polynucleotide of the active moiety is an antisense strand complementary to any region of a non-target protein-coding transcript, for example, a non-target-coding RNA. In one embodiment of the present invention, the non-target-coding RNA is human or mouse metastasis-associated lung adenocarcinoma transcript 1 (MALAT1).

In one embodiment of the present invention, the polynucleotide of (i) the active moiety is a polynucleotide selected from the following polynucleotides (a) to (d):
(a) a polynucleotide having the base sequence shown in any one of SEQ ID NOS: 1, 6 to 12, and 21;
(b) a polynucleotide having 70% or more sequence identity to the polynucleotide (a);
(c) a polynucleotide wherein a few nucleotides are substituted, deleted, added, and/or inserted in the polynucleotide (a); and
(d) a polynucleotide containing any of the polynucleotides (a) to (c) as a partial sequence.

The polynucleotide (b) has 70% or more, preferably 80% or more, more preferably 85% or more, still more preferably 90% or more, and particularly preferably 95% or more, for example, 96% or more, 97% or more, 98% or more, 99% or more, or 99.5% or more, sequence identity to the base sequence of the polynucleotide (a). Preferably, the polynucleotide (b) has an activity of suppressing the expression of a target transcript.

The polynucleotide (c) may be a polynucleotide wherein a few, preferably one to several, for example, 1, 2, 3, 4, 5, 6, 7, 8, 9, or 10, nucleotides are substituted, deleted, added, and/or inserted in the polynucleotide (a). Preferably, the polynucleotide (c) has an activity of suppressing the expression of a target transcript.

The polynucleotide (d) is a polynucleotide containing any of the polynucleotides (a) to (c) as a partial sequence, and preferably has an activity of suppressing the expression of a target transcript. In one embodiment of the present invention, the polynucleotide (d) is 8 to 100 bases in length. Preferably, the length is at least 10 bases, at least 12 bases, or at least 13 bases. The length may be preferably 100 bases or less, 35 bases or less, 25 bases or less, or 20 bases or less.

As used herein, the "sequence identity" concerning base sequences is a value expressed in percentage (%) that is determined by aligning two base sequences to be compared such that as many bases as possible match between the base sequences, and then dividing the number of matching bases by the total number of bases. During the alignment, gaps are inserted, as required, into one or both of the two sequences to be compared. This sequence alignment may be performed using a well-known program such as BLAST, FASTA, or CLUSTAL W, for example (Karlin and Altschul, Proc. Natl. Acad. Sci. U.S.A., 87: 2264-2268, 1993; and Altschul et al., Nucleic Acids Res., 25: 3389-3402, 1997). If gaps are inserted, the total number of bases represents the number of bases determined when one gap is counted as one base. If the total number of bases thus counted differs between the two sequences to be compared, the sequence identity (%) is calculated by dividing the number of matching bases by the total number of bases of the longer sequence.

In general, the presence of the above-described modifications or nucleotide analogs has no effect on the sequence identity.

In one embodiment of the present invention, the polynucleotide of (ii) the carrier moiety is a polynucleotide selected from the following polynucleotides (a') to (d'):
(a') a polynucleotide having the base sequence shown in any one of SEQ ID NOS: 2 to 5, 13 to 20, and 22;
(b') a polynucleotide having 70% or more sequence identity to the polynucleotide (a');
(c') a polynucleotide wherein a few nucleotides are substituted, deleted, added, and/or inserted in the polynucleotide (a'); and
(d') a polynucleotide containing any one of the polynucleotides (a') to (c') as a partial sequence.

The polynucleotide (b') has 70% or more, preferably 80% or more, more preferably 85% or more, still more preferably 90% or more, and particularly preferably 95% or more, for example, 96% or more, 97% or more, 98% or more, 99% or more, or 99.5% or more, sequence identity to the base sequence of the polynucleotide (a'). Preferably, the polynucleotide (b') has a function as a carrier of the active moiety.

The polynucleotide (c') may be a polynucleotide wherein a few, preferably one to several, for example, 1, 2, 3, 4, 5, 6, 7, 8, 9, or 10, nucleotides are substituted, deleted, added, and/or inserted in the polynucleotide (a'). Preferably, the polynucleotide (c') has a function as a carrier of the active moiety.

The polynucleotide (d') is a polynucleotide containing any of the polynucleotides (a') to (c') as a partial sequence, and preferably has a function as a carrier of the active moiety. In one embodiment of the present invention, the polynucleotide (d') is 8 to 100 bases in length. Preferably, the length is at least 10 bases, at least 12 bases, at least 13 bases, at least 14 bases, at least 15 bases, or at least 16 bases. The length may be preferably 100 bases or less, 45 bases or less, 35 bases or less, 30 bases or less, 25 bases or less, 24 bases or less, 23 bases or less, 22 bases or less, 21 bases or less, or 20 bases or less.

In one embodiment of the present invention, the nucleic acid complex preferably comprises a functional moiety in the carrier moiety. In one embodiment of the present invention, the functional moiety may be bound to the carrier moiety, preferably to the polynucleotide of the carrier moiety. In one embodiment of the present invention, the functional moiety may be bound to the bulge. The bonding between the polynucleotide and the functional moiety may be direct bonding or indirect bonding via another substance. In a certain embodiment, however, the functional moiety is preferably directly bound to the polynucleotide of the carrier moiety by covalent bonding, ionic bonding, or hydrogen bonding, for example, and more preferably by covalent bonding, from the viewpoint of achieving more stable bonding.

In a certain embodiment, the "functional moiety" is not particularly limited in terms of structure, so long as the functional moiety provides a desired function for the nucleic acid complex, the carrier moiety, and/or the polynucleotide of the carrier moiety comprising it or to which it is bound. Examples of desired functions include a labeling function, a purification function, and a targeted delivery function. Examples of moieties that provide the labeling function include compounds such as fluorescent proteins and luciferase. Examples of moieties that provide the purification function include compounds such as biotin, avidin, His-tagged peptides, GST-tagged peptides, and FLAG-tagged peptides.

Furthermore, in one embodiment of the present invention, the nucleic acid complex may comprise, as the functional moiety, a molecule having an activity of delivering the complex to a target site, from the viewpoint of allowing the active moiety to be delivered to the target site with high specificity and efficiency, and suppressing the expression of a target gene by means of the active moiety very effectively. Preferably, the carrier moiety comprises the molecule, and the molecule is preferably bound to the polynucleotide of the carrier moiety, more preferably to the bulge structure.

The moiety having the "targeted delivery function" may be a lipid, for example, from the viewpoint of allowing the nucleic acid complex in a certain embodiment to be delivered to the liver or the like with high specificity and efficiency, for example. Examples of such lipids include lipids such as cholesterol and fatty acids (for example, vitamin E (tocopherols and tocotrienols), vitamin A, and vitamin D), lipid-soluble vitamins such as vitamin K (for example, acylcarnitine), intermediate metabolites such as acyl-CoA, glycolipids, glycerides, and derivatives thereof. In particular, it is preferred to use cholesterol and vitamin E (tocopherols and tocotrienols) in a certain embodiment, from the viewpoint of having higher safety. Moreover, examples of the "functional moiety" in a certain embodiment include sugars (for example, glucose and sucrose), from the viewpoint of allowing the nucleic acid of the present invention to be delivered to the brain with high specificity and efficiency. Furthermore, examples of the "functional moiety" also include small molecules and biomolecules/bioactive molecules (hereinafter also referred to as "small-molecule ligands", such as anisamide, tirofiban, and 2-pyrrolidin-1-yl-N-[4-[4-(2-pyrrolidin-1-yl-acetylamino)-benzyl]-phenyl]-acetamide). Examples of the "functional moiety" in a certain embodiment further include peptides or proteins such as receptor ligands, antibodies and/or fragments thereof, as well as folic acid, from the viewpoint of allowing the nucleic acid complex in a certain embodiment to be delivered to various organs with high specificity and efficiency, by binding to various proteins present on the cell surface of the various organs. The "peptide" is a substance in which two or more molecules of amino acids are linked by peptide bonds, and herein refers to a substance in which less than 100 residues of amino acids are linked. In the present invention, a peptide in which preferably 60 or less residues of amino acids, for example, 50 or less, 40 or less, 30 or less, 20 or less, or 10 or less residues of amino acids, for example, 9 or less, 8 or less, 7 or less, 6 or less, or 5 or less residues of amino acids are linked is used as the moiety having the targeted delivery function (delivery functional moiety). Examples of the peptide to be used as the delivery functional moiety (hereinafter also referred to as the "peptide ligand") include, although not particularly limited to, cyclic RGD sequence-containing peptides, insulin, glucagon-like peptide-1, vasopressin, and oxytocin.

As used herein, the "cyclic RGD sequence-containing peptides" (hereinafter also referred to as "cRGD" or "cRGD peptides") refers to peptides that have at least one arginine-glycine-aspartic acid (RGD) sequence, and form a cyclic structure. It is known that an RGD sequence binds to integrin molecules (particularly αᵥβ₃, αᵥβ₅, and the like), which are cell adhesion molecules on the cell surface, to activate them, or induces endocytosis in cells. The use of such an RGD peptide as a tumor-targeting ligand is also known. In the present invention, any cRGD peptide that has such an RGD sequence and forms a cyclic structure may be used. The cRGD peptide is not particularly limited in sequence length; however, from the viewpoint of forming a cyclic structure, the cRGD peptide generally contains 3 or more amino acids, and preferably 4 to 15 or 5 to 10 amino acids, for example.

In the present invention, as with the "peptide ligand", the above-described lipids, proteins, sugar chains, small molecules, and biomolecules/bioactive molecules may also be referred to as "lipid ligands", "protein ligands", "sugar chain ligands", "small molecule ligands", and "biomolecule/bioactive molecule ligands", respectively, or these ligands may also be simply referred to as "ligands" collectively.

In one embodiment of the present invention, (ii) the carrier moiety comprises the functional moiety (delivery functional moiety) having the targeted delivery function, wherein the delivery functional moiety is preferably a molecule selected from the group consisting of lipids, peptides, proteins, sugar chains, small molecules, and biomolecules/bioactive molecules, and the delivery functional moiety is, for example, a peptide containing a cyclic arginine-glycine-aspartic acid (RGD) sequence, N-acetylgalactosamine, cholesterol, vitamin E (tocopherol), stearic acid, docosanoic acid, anisamide, folic acid, anandamide, or spermine.

In one embodiment of the present invention, (ii) the carrier moiety consists of the polynucleotide and the functional moiety, particularly the delivery functional moiety.

In one further embodiment of the present invention, (ii) the carrier moiety is free of the functional moiety, particularly the delivery functional moiety.

In another embodiment of the present invention, the present invention relates to a double-stranded nucleic acid complex comprising:
(i) an active moiety comprising a polynucleotide comprising, as structural units, at least four contiguous phosphorothioated deoxyribonucleotides, a 5'-wing region that is positioned 5' to the at least four contiguous deoxyribonucleotides, and consists of two to five phosphorothioated bridged nucleotides, and a 3'-wing region that is positioned 3' to the at least four contiguous deoxyribonucleotides, and consists of two to five phosphorothioated bridged nucleotides; and
(ii) a carrier moiety comprising a polynucleotide consisting of one or more unmodified ribonucleotides and one or more unmodified deoxyribonucleotides as structural units, the polynucleotide being at least partially complementary to the polynucleotide of (i) the active moiety, wherein
   the polynucleotide of (ii) the carrier moiety comprises at least one bulge, and the bulge is formed of one or more unmodified ribonucleotides,
   the polynucleotide of (i) the active moiety comprises no mismatch compared to the polynucleotide of (ii) the carrier moiety and/or a target transcript, and
(ii) the carrier moiety does not comprise a functional moiety having a targeted delivery function.

For example, the nucleic acid complex of the present invention having a ligand as described above may be targeted to a target cell or tissue having (on the surface, for example) a receptor to which the ligand can specifically bind. The type of the target cell or tissue is not particularly limited, and any cell or tissue suitable for the purpose or the like may be used as the target. For example, the target cell may be a cell that forms a tissue or organ. Alternatively, the target cell may be a cell that exists independently, such as a leukemic cell, or may be a cell that forms a tumor in a tissue, such as a solid cancer cell, or a cell that infiltrates lymphoid tissue or another tissue (these cells are also simply referred to as "cancer cells" collectively, hereinafter).

In one embodiment of the present invention, the ligand may be bound directly, or bound indirectly via a linker, to the nucleic acid complex, preferably to the carrier moiety, and more preferably to the polynucleotide of the carrier moiety. For example, the ligand may be bound directly or indirectly via a linker to the 5' end or 3' end, preferably the 5' end, of the polynucleotide of the carrier moiety. Methods for binding the ligand to the polynucleotide optionally via a linker are well known to those skilled in the art. A person skilled in the art can achieve the above-described bonding by selecting a known method suitable for the type of the ligand or linker to be used.

In one embodiment, the delivery functional moiety consists of a ligand only. In a further embodiment of the present invention, the delivery functional moiety consists of a ligand and a linker only.

In general, when a compound is enterally administered (orally administered, for example), the compound is diffused in the body through the lymphatic vessels, rather than the blood vessels. However, the compound needs to have a molecular weight of 11000 to 17000 daltons or more to reach the lymphatic vessels. Furthermore, because the enterally administered compound will be exposed to RNase A in the intestine, a nucleic acid drug containing ribonucleotides preferably have all the ribonucleotides modified by 2'-O-methylation, for example. Thus, in one embodiment of the present invention, if the nucleic acid complex comprises ribonucleotides, or modified nucleotides and/or nucleotide analogs having an RNA structure in terms of structural chemistry, such as LNAs, then all these structural units are 2'-O-methylated.

Typical examples of suitable nucleic acid complexes in some embodiments have been described above; however, the nucleic acid complexes in some embodiments are not limited to these typical examples. Furthermore, in some embodiments, a person skilled in the art can prepare the polynucleotide of the active moiety and the polynucleotide of the carrier moiety, by selecting a known method, as appropriate. For example, nucleic acids can be prepared by designing the respective base sequences of the nucleic acids on the basis of the information of the base sequence of a target transcript (or, in some cases, the base sequence of a target gene), synthesizing the nucleic acids with a commercially available automated nucleic acid synthesizer (from Applied Biosystems, Inc. or Beckman Coulter, Inc., for example), and subsequently purifying the resulting polynucleotides with a reverse phase column, for example. The nucleic acids thus prepared are then mixed in an appropriate buffer solution and denatured at about 90 to 98°C for several minutes (for example, for 5 minutes), and then annealed at about 30 to 70°C for about 1 to 8 hours. As a result, nucleic acid complexes in some embodiments can be prepared. Furthermore, a nucleic acid complex to which a functional moiety is bound can be prepared by using a nucleic acid species to which a functional moiety has been bound in advance, and performing synthesis, purification, and annealing as described above. Many methods for binding functional moieties to nucleic acids are well known in the art.

As described above, the nucleic acid complex of the present invention is believed to exhibit the antisense effect, mainly via the RNase H-dependent pathway. In a further embodiment of the present invention, however, the nucleic acid complex of the present invention can have an RNase H-independent antisense effect. The "RNase H-independent antisense effect" refers to the activity of suppressing the expression of a target gene attributable to the inhibition of translation or a splicing function-modifying effect such as exon skipping, which is induced by hybridization between a transcript of a target gene (RNA sense strand) and a nucleic acid strand complementary to a partial sequence thereof.

### <Compositions for suppressing the expression of target genes or target transcripts>

Nucleic acid complexes in some embodiments can be delivered to target sites with high specificity and efficiency, and can suppress the expression of target genes or the levels of target transcripts very effectively, as described in the Examples below. Thus, the present invention can provide, for example, compositions for suppressing the expression of target genes by means of the antisense effect, the compositions comprising nucleic acid complexes in some embodiments as active ingredients. In particular, nucleic acid complexes in some embodiments can achieve high efficacy even when administered at low concentrations, and can exhibit reduced side effects by suppressing the distribution of the antisense nucleic acid in organs other than the target delivery region. Thus, some embodiments can provide pharmaceutical compositions for treating or preventing diseases associated with increased expression of target genes, such as metabolic diseases, tumors, and infections.

Thus, in one embodiment of the present invention, the present invention relates to a pharmaceutical composition comprising the nucleic acid complex and optionally a pharmacologically acceptable carrier.

In an alternative embodiment of the present invention, the present invention relates to use of the nucleic acid complex for the manufacture of a pharmaceutical composition.

In a further alternative embodiment, the present invention relates to a method for treating cancer in a mammal, comprising the step of administering the nucleic acid complex to the mammal.

The pharmaceutical composition may be a pharmaceutical composition for suppressing the proliferation of cancer cells, or may be a pharmaceutical composition for treating and/or preventing cancer.

The cancer is selected from the group consisting of, for example, brain tumor; squamous cell carcinoma of the head, neck, lung, uterus, or esophagus; melanoma; adenocarcinoma of the lung or uterus; renal carcinoma; malignant mixed tumor; hepatoma; basal cell carcinoma; acanthoma-like gingival tumor; intraoral tumor; perianal adenocarcinoma; anal sac tumor; anal sac apocrine adenocarcinoma; sertoli cell tumor; vaginal vestibule carcinoma; sebaceous adenocarcinoma; sebaceous gland epithelioma; sebaceous adenoma; sweat gland carcinoma; intranasal adenocarcinoma; nasal gland carcinoma; thyroid carcinoma; colorectal carcinoma; bronchial gland carcinoma; adenocarcinoma; ductal carcinoma; breast adenocarcinoma; mixed breast adenocarcinoma; malignant mixed mammary tumor; intraductal papillary adenocarcinoma; fibrosarcoma; hemangiopericytoma; sarcoma; osteosarcoma; chondrosarcoma; soft tissue sarcoma; histiocytic sarcoma; myxosarcoma; undifferentiated sarcoma; lung carcinoma; mastocytoma; cutaneous leiomyoma; intraabdominal leiomyoma; leiomyoma; chronic lymphocytic leukemia; lymphoma; gastrointestinal lymphoma; digestive organ lymphoma; small cell or medium cell lymphoma; adrenomedullary tumor; granulosa cell tumor; pheochromocytoma; head and neck carcinoma; breast carcinoma; lung carcinoma; colon carcinoma; ovarian carcinoma; prostatic carcinoma; neuroglioma; glioblastoma; spongiocytoma; polymorphism glioblastoma; inflammatory breast carcinoma; Wilms tumor; Ewing's sarcoma; rhabdomyosarcoma; ependymoma; medulloblastoma; renal carcinoma; hepatoma; melanoma; pancreatic carcinoma; osteoclastoma; thyroid carcinoma; lymphoblastic T cell leukemia; chronic myeloid leukemia; chronic lymphocytic leukemia; hairy-cell leukemia; acute lymphoblastic leukemia; acute myeloid leukemia; AML; chronic neutrophilic leukemia; acute lymphoblastic T cell leukemia; plasmocytoma; immunoblastic large-cell leukemia; mantle cell leukemia; multiple myeloma; megakaryoblastic leukemia; acute megakaryocytic leukemia; promyelocytic leukemia; erythroleukemia; Hodgkins lymphoma; non-Hodgkins lymphoma; lymphoblastic T cell lymphoma; Burkitt's lymphoma; follicular lymphoma; neuroblastoma; bladder carcinoma; urothelial carcinoma; vulvar carcinoma; cervical carcinoma; endometrial carcinoma; mesothelioma; esophageal carcinoma; salivary gland carcinoma; hepatocellular carcinoma; gastric carcinoma; nasopharyngeal carcinoma; buccal carcinoma; intraoral carcinoma; GIST (gastrointestinal stromal tumor); epidermoid carcinoma; alveolar basal epithelial adenocarcinoma; and testis carcinoma.

In a further embodiment of the present invention, the present invention relates to a method for reducing the level of a transcript in a cell, comprising the step of contacting the nucleic acid complex with the cell.

In an alternative embodiment of the present invention, the present invention relates to a method for suppressing the proliferation of a cell, preferably a cancer cell, comprising the step of contacting the nucleic acid complex with the cell.

In an alternative embodiment of the present invention, the present invention relates to use of the nucleic acid complex for reducing the expression of a target gene or a non-protein-coding transcript, for example, a non-coding RNA, in a mammal.

In an alternative embodiment of the present invention, the present invention relates to use of the nucleic acid complex for suppressing the proliferation of a target cell, preferably a cancer cell, preferably in a target tissue or target site, in a mammal.

The present invention also relates to use of the nucleic acid complex for the manufacture of a medicament for reducing the expression of a target gene or a non-protein-coding transcript (for example, a non-coding RNA) in a mammal. The present invention also relates to use of the nucleic acid complex for the manufacture of a medicament for suppressing the proliferation of a target cell, preferably a cancer cell, preferably in a target tissue or target site, in a mammal.

In a further embodiment, the present invention relates to a method for reducing the expression level of a target gene or non-protein-coding transcript (for example, a non-coding RNA) in a mammal, comprising the step of administering the nucleic acid complex to the mammal. In an alternative embodiment of the present invention, the present invention relates to a method for suppressing the proliferation of a target cell, preferably a cancer cell, preferably in a target tissue or target site, in a mammal, comprising the step of administering the nucleic acid complex to the mammal. In the above-described embodiment, preferably, the polynucleotide of the active moiety is an antisense strand complementary to any region of a transcript, for example, mRNA of a target gene or a non-protein-coding transcript (for example, a non-coding RNA).

In one embodiment, the transcript is a protein-coding mRNA transcript. Preferably, the protein is human BCL2, human STAT3. In another embodiment, the transcript is a non-protein-coding transcript, for example, the non-coding RNA mouse metastasis-associated lung adenocarcinoma transcript 1 (MALAT1) (GenBank Accession number NR_002847).

Furthermore, in one embodiment of the present invention, the target gene is human bcl-2 or human STAT3.

In one preferred embodiment, the mammal is a human.

The composition or medicament comprising the nucleic acid complex may be formulated using known pharmaceutical methods, and may be formulated into desired forms of administration or dosage forms, for example. The composition or medicament may be used enterally (orally, for example) in the form of capsules, tablets, pills, liquids, powders, granules, fine granules, film-coating agents, pellets, troches, sublingual agents, peptizers, buccal preparations, pastes, syrups, suspensions, elixirs, emulsions, coating agents, ointments, plasters, cataplasms, transdermal preparations, lotions, inhalers, aerosols, injections, suppositories, or the like, or may be used non-enterally.

In the formulation of the composition or medicament, pharmacologically acceptable carriers or carriers acceptable as foods and drinks, specifically sterilized water, physiological saline, vegetable oils, solvents, bases, emulsifiers, suspending agents, surfactants, pH adjusting agents, stabilizers, flavors, fragrances, excipients, vehicles, antiseptics, binders, diluents, isotonizing agents, soothing agents, extending agents, disintegrants, buffering agents, coating agents, lubricating agents, colorants, sweetening agents, thickening agents, corrigents, dissolution aids, and other additives, may be incorporated, as appropriate.

For example, in the formulation of the composition or medicament, as disclosed in "Kazutaka Nishina et al., Molecular Therapy, Vol. 16, pp. 734-740, 2008", double-stranded nucleic acid complexes in some embodiments to which lipids are bound as functional moieties may be caused to form complexes with lipoproteins such as chylomicron or chylomicron remnant. Furthermore, from the viewpoint of increasing the efficiency of enteral administration, complexes (mixed micelles and emulsions) with substances having colonic mucosal epithelial permeability-enhancing action (for example, medium-chain fatty acids, long-chain unsaturated fatty acids, or derivatives thereof (salts, ester forms or ether forms)) and surfactants (nonionic surfactants and anionic surfactants), in addition to the lipoproteins, may be formed.

Examples of preferred modes of administration of the composition or medicament include, although not particularly limited to, enteral (oral, for example) or non-enteral administration, more specifically, intravenous administration, intraarterial administration, intraperitoneal administration, subcutaneous administration, intracutaneous administration, tracheobronchial administration, rectal administration, intramuscular administration, and administration by transfusion.

In each of the embodiments, the nucleic acid complex or composition may be used for animals including humans as subjects. That is, humans and non-human animals may be used as subjects. Non-human animals are not particularly limited, and various domestic animals, poultry, pets, experimental animals, and the like may be used as subjects.

When any of the compositions in some embodiments is administered or ingested, the dose or the amount of ingestion of the composition may be selected as appropriate, in accordance with the age, body weight, symptoms, and health condition of the subject, as well as the type of the composition (a pharmaceutical product, a food or drink, or the like), for example. The effective amount of ingestion of a composition according to a certain embodiment is preferably 0.001 to 50 mg/kg/day, calculated as nucleotides.

The double-stranded nucleic acid of the present invention can be delivered to a target site with high specificity and efficiency, can suppress the expression of a target gene or the level of a target transcript very effectively, as described in the Examples below, and can exhibit an antitumor effect. Thus, one embodiment of the present invention can provide a method for suppressing the expression of a target gene or a target transcript by means of the antisense effect, by administering the nucleic acid complex to a subject. The present invention can further provide a method for treating or preventing various diseases associated with, for example, increased expression of a target gene, by administering the composition of the present invention to a subject.

### [Examples]

The present invention will be more specifically described hereinafter with examples and comparative examples, although the embodiments are not limited to the following examples.

### Example 1

### [Synthesis of double-stranded nucleic acids]

On the basis of the sequence of human BCL2 gene, double-stranded nucleic acids having a basic backbone of DNA-bulge-type DNA targeted to the gene were designed. As specific examples of the double-stranded nucleic acids targeted to BCL2 gene, an antisense-strand nucleotide sequence (16-mer) is shown in SEQ ID NO: 1, and complementary-strand nucleotide sequences are shown in SEQ ID NOS: 2 to 5. For each of the gene sequences, the antisense strand and the respective complementary strand were annealed to form a double-stranded nucleic acid. A double-stranded nucleic acid formed between SEQ ID NOS: 1 and 2 is designated as BRDB-1; a double-stranded nucleic acid formed between SEQ ID NOS: 1 and 3 is designated as BRDB-2; a double-stranded nucleic acid formed between SEQ ID NOS: 1 and 4 is designated as BRDB-3; and a double-stranded nucleic acid formed between SEQ ID NOS: 1 and 5 is designated as BRDB-4. The synthesis of these double-stranded nucleic acids was commissioned to GeneDesign, Inc. In the sequences, the underlined nucleotides denote RNAs; 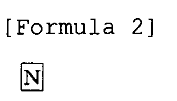 ; and the others denote DNAs. As forms of nucleotide linkages, "ps" denotes a thiophosphate linkage, and "po" denotes a phosphodiester linkage.

**[Table 1]**

| |
|---|
| SEQ ID NO: 1: |
| 5'- CpsTpsCpsCpsCpsApsGpsCpsGpsTpsGpsCpsGpsCpsCpsA -3' |
| SEQ ID NO: 2: |
| 5'-TpoGpoGpoCpoGpoCpoApoCpoUpoGpoCpoTpoGpoGpoGpoApoG-3' |
| SEQ ID NO: 3: |
| 5'-TpoGpoGpoCpoGpoCpoApoCpoUpoUpoGpoCpoTpoGpoGpoGpoApoG-3' |
| SEQ ID NO: 4: |
| 5'-TpoGpoGpoCpoGpoCpoApoCpoUpoUpoUpoGpoCpoTpoGpoGpoGpoApoG-3' |
| SEQ ID NO: 5: |
| 5'-TpoGpoGpoCpoGpoCpoApoCpoUpoUpoUpoUpoGpoCpoTpoGpoGpoGpoApoG-3' |

### Example 2

### [Cell culture]

As human cancer-derived cell lines for use in the below-described experiments, cell lines maintained in the following manners were used.

Human epidermoid carcinoma-derived cell line (A431 cell line, purchased from the JCRB Cell Bank, cell number: JCRB0004) was maintained in 5 mass% CO₂ at 37°C in growth medium (DMEM from GIBCO) supplemented with 10 mass% fetal bovine serum, 100 units/ml of penicillin, and 100 µg of streptomycin, and human pancreatic carcinoma-derived cell line (AsPC-1 cell line, purchased from the ATCC Cell Bank, cell number: CRL-1682) was maintained in 5 mass% CO₂ at 37°C in growth medium (RPMI1640 from GIBCO) supplemented with 10 mass% fetal bovine serum, 100 units/ml of penicillin, and 100 µg of streptomycin.

In addition, human alveolar basal epithelial adenocarcinoma-derived cell line (A549 cell line, purchased from the JCRB Cell Bank, cell number: JCRB0076) and human colorectal carcinoma-derived cell line (HCT116 cell line, purchased from the ATCC Cell Bank, cell number: CCL-247) were maintained in 5 mass% CO₂ at 37°C in growth medium (MEM from GIBCO) supplemented with 10 mass% fetal bovine serum, MEM non-essential amino acids (NEAA), 100 units/ml of penicillin, and 100 µg of streptomycin.

### Example 3

### [Cell proliferation assay]

The cell proliferation assay performed in the below-described experiments was carried out in the following manner, unless otherwise indicated. Cells were cultured in 100 µl/well of the medium in 96-well microplates under humid conditions (5%CO₂ at 37°C). In the cell proliferation assay, 10 µl per well of the cell proliferation reagent WST-1 (Roche) was added, and a microplate reader (Bio Rad) was used to measure absorbance.

### Example 4

### [Quantitative RT-PCR]

Quantitative RT-PCR performed in the below-described experiments was carried out in the following manner, unless otherwise indicated. Total RNA was extracted from cultured cells with the Rneasy Mini Kit (QIAGEN). Quantitative RT-PCR using the total RNA was performed with the Quant-Fast Probe RT-PCR Kit (QIAGEN) under the recommended conditions. TaqMan Gene Expression Assays probes from Applied Biosystem were used as primers. β-Actin from Applied Biosystem was used as endogenous control primers. Amplification in quantitative RT-PCR was performed with ROTOR-Gene Q (QIAGEN). mRNA expression levels were calculated using the Delta-Delta CT method.

### Example 5

[Cell proliferation-suppressing activity tests on the human bcl2 gene-targeted double-stranded nucleic acids]

The following experiments were performed to investigate *in vitro* cell proliferation-suppressing activities of the human BCL2 gene-targeted double-stranded nucleic acids. Initially, the double-stranded nucleic acids (four in total) obtained by annealing SEQ ID NO: 1 and SEQ ID NOS: 2 to 5 were prepared. Next, A431, AsPC-1, A549, and HCT116 cell lines were transfected with 10 nM each of these double-stranded nucleic acids using Lipofectamine 2000 (Invitrogen). The transfected cell lines were cultured for 72 hours after the transfection, and then the cell proliferation-suppressing activities of the double-stranded nucleic acids were measured in accordance with the cell proliferation assay method described in Example 3 above.

In addition, the cell proliferation assay was similarly performed with a double-stranded nucleic acid targeted to a non-target gene (GL3), instead of the above-described double-stranded nucleic acids.

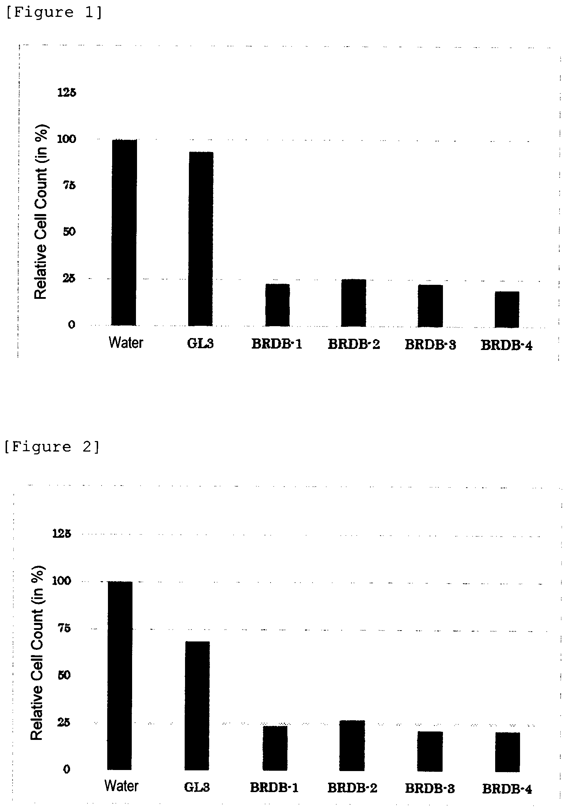

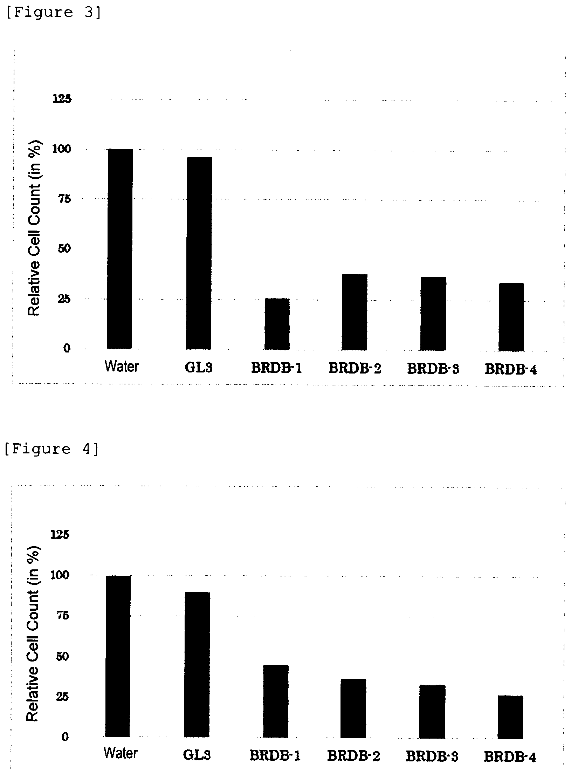

The results for A431 cell line are shown in Figure 1, the results for AsPC-1 cell line are shown in Figure 2, the results for A549 cell line are shown in Figure 3, and the results for HCT116 cell line are shown in Figure 4. As seen from Figures 1 to 4, the double-stranded nucleic acids having a basic backbone of DNA-bulge-type DNA targeted to human BCL2 gene exhibited proliferation-suppressing activities against the various types of cancer cell lines.

### Example 6

### [mRNA expression-suppressing activity tests on human BCL2 gene-targeted double-stranded nucleic acids]

The following experiments were performed to investigate *in vitro* mRNA expression-suppressing activities of human BCL2 gene-targeted double-stranded nucleic acids. Initially, the double-stranded nucleic acids obtained by annealing SEQ ID NO: 1 and SEQ ID NO: 5 were prepared. Next, A431, AsPC-1, A549, and HCT116 cell lines were transfected with 10 nM each of these double-stranded nucleic acids using Lipofectamine 2000 (Invitrogen). The transfected cell lines were cultured for 24 hours after the transfection, and then the mRNA expression-suppressing activities of the double-stranded nucleic acids were quantified in accordance with the quantitative RT-PCR method described in Example 4 above.

In addition, the quantification of mRNA expression-suppressing activity was similarly performed with a double-stranded nucleic acid targeted to a non-target gene (GL3), instead of the above-described double-stranded nucleic acids.

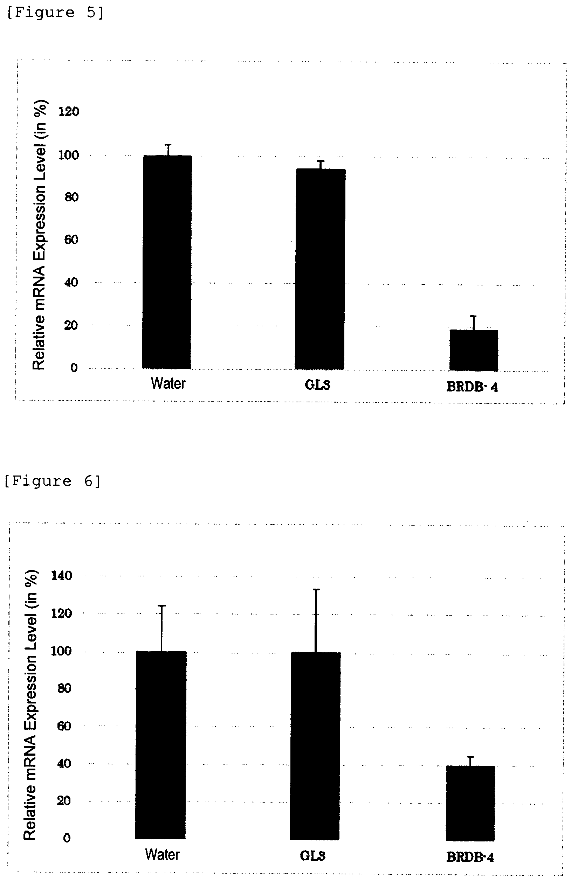

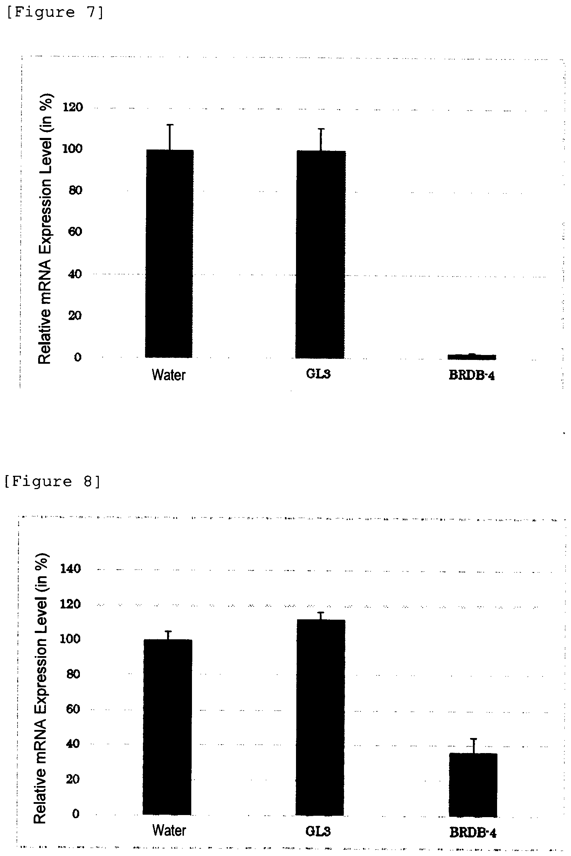

The results for A431 cell line are shown in Figure 5, the results for AsPC-1 cell line are shown in Figure 6, the results for A549 cell line are shown in Figure 7, and the results for HCT116 cell line are shown in Figure 8. As seen from Figures 5 to 8, the double-stranded nucleic acids having a basic backbone of DNA-bulge-type DNA targeted to human BCL2 gene exhibited mRNA expression-suppressing activities against the various types of cancer cell lines.

### Example 7

### [Synthesis of double-stranded nucleic acids]

On the basis of the sequences of human BCL2 and MALAT1 genes, double-stranded nucleic acids having a basic backbone of DNA-bulge-type DNA targeted to these genes were designed. As specific examples, antisense-strand nucleotide sequences targeted to BCL2 gene are shown in SEQ ID NOS: 6 to 12, an antisense-strand nucleotide sequence targeted to MALAT1 gene is shown in SEQ ID NO: 21, complementary-strand nucleotide sequences targeted to BCL2 gene are shown in SEQ ID NOS: 13 to 20, and a complementary-strand nucleotide sequence targeted to MALAT1 gene is shown in SEQ ID NO: 22 (Table 2). For each of the gene sequences, the antisense strand and the respective complementary strand were annealed to form a double-stranded nucleic acid. A double-stranded nucleic acid formed between SEQ ID NOS: 1 and 13 is designated as BRDB-5; a double-stranded nucleic acid formed between SEQ ID NOS: 1 and 14 is designated as BRDB-6; a double-stranded nucleic acid formed between SEQ ID NOS: 1 and 15 is designated as BRDB-7; a double-stranded nucleic acid formed between SEQ ID NOS: 1 and 16 is designated as BRDB-8; a double-stranded nucleic acid formed between SEQ ID NOS: 1 and 17 is designated as BRDB-9; a double-stranded nucleic acid formed between SEQ ID NOS: 1 and 18 is designated as BRDB-10; a double-stranded nucleic acid formed between SEQ ID NOS: 1 and 19 is designated as BRDB-11; double-stranded nucleic acids formed between SEQ ID NO: 6 and SEQ ID NOS: 2 to 5, 13, and 14 are designated as BRD-12 to 17; double-stranded nucleic acids formed between SEQ ID NO: 7 and SEQ ID NOS: 2 to 5, 13, and 14 are designated as BRD-18 to 23; double-stranded nucleic acids formed between SEQ ID NOS: 8 to 12, and SEQ ID NO: 5 are designated as BRD-24 to 28; a double-stranded nucleic acid formed between SEQ ID NOS: 1 and 20 is designated as BRD-29; and a double-stranded nucleic acid formed between SEQ ID NOS: 21 and 22 is designated as BRD-30.

5'-Octyl-tochopherol (Link Technology) denoted by the following symbol was bound to the 5' end of each of the sequences of SEQ ID NOS: 20 and 22:

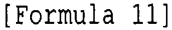

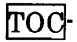

The synthesis of these double-stranded nucleic acids was commissioned to GeneDesign, Inc.

In the sequences, "I" denotes inosine or deoxyinosine; the underlined nucleotides denote RNAs; the following symbol denotes an LNA: 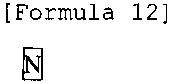 ; and the others denote DNAs. As forms of nucleotide linkages, "ps" denotes a thiophosphate linkage, and "po" denotes a phosphodiester linkage. "N(f)" denotes a 2'-F modified nucleotide, and "X" denotes 1,2-dideoxy-B-D-ribose-3-CEP (Chemgene; Cat No. ANP-7058).

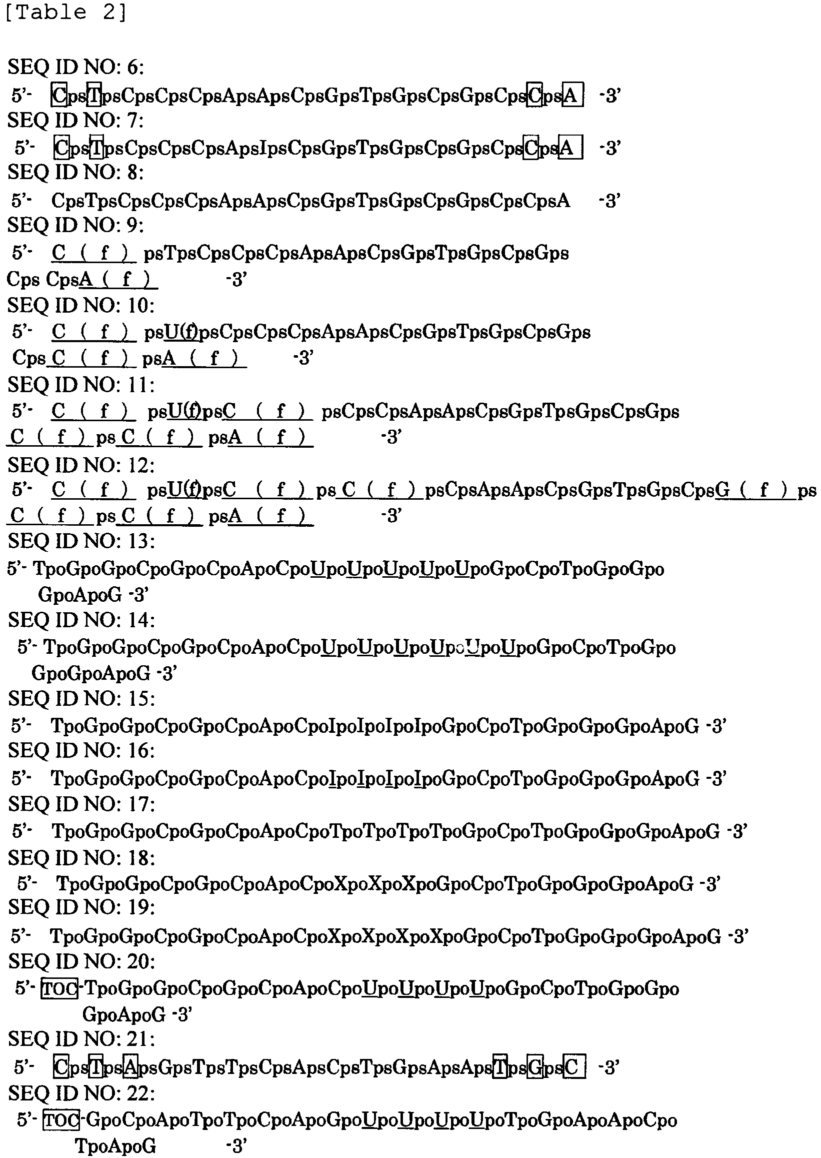

### Example 8

### [Cell culture]

As human cancer-derived cell lines for use in the below-described experiments, cell lines maintained in the following manners were used.

Human hepatoma-derived cell line (HepG2 cell line, purchased from the Riken Cell Bank, cell number: RBRC-RCB1886) and human pancreatic carcinoma-derived cell line (HPAC cell line, purchased from the ATCC Cell Bank, cell number: CRL-2119) were maintained in 5 mass% CO₂ at 37°C in growth medium (DMEM from GIBCO) supplemented with 10 mass% fetal bovine serum, 100 units/ml of penicillin, and 100 µg of streptomycin; human gastric carcinoma-derived cell line (MKN45 cell line, purchased from the JCRB Cell Bank, cell number: JCRB0254), human pancreatic carcinoma-derived cell line (PANC-1 cell line, purchased from the Riken Cell Bank, cell number: RBRC-RCB2095), human prostatic carcinoma-derived cell line (DU145 cell line, purchased from the Riken Cell Bank, cell number: RBRC-RCB2143), human prostatic carcinoma-derived cell line (LNCap cell line, purchased from the Riken Cell Bank, cell number: RBRC-RCB2144), human prostatic carcinoma-derived cell line (PC-3 cell line, purchased from the Riken Cell Bank, cell number: RBRC-RCB2145), and human ovarian carcinoma-derived cell line (OVCAR-3 cell line, purchased from the Riken Cell Bank, cell number: RBRC-RCB2135) were maintained in 5 mass% CO₂ at 37°C in growth medium (RPMI1640 from GIBCO) supplemented with 10 mass% fetal bovine serum, 100 units/ml of penicillin, and 100 µg of streptomycin; human renal carcinoma-derived cell line (Caki-1 cell line, purchased from the JCRB Cell Bank, cell number: JCRB0801), human gastric carcinoma-derived cell line (HGC-27 cell line, purchased from the Riken Cell Bank, cell number: RBRC-RCB0500), human bladder carcinoma-derived cell line (T24 cell line, purchased from the Riken Cell Bank, cell number: RBRC-RCB2536), and human colorectal carcinoma-derived cell line (LS174T cell line, purchased from the ATCC Cell Bank, cell number: CRL-188) were maintained in 5 mass% CO₂ at 37°C in growth medium (MEM from GIBCO) supplemented with 10 mass% fetal bovine serum, MEM non-essential amino acids (NEAA), 100 units/ml of penicillin, and 100 µg of streptomycin; human breast adenocarcinoma-derived cell line (MCF-7 cell line, purchased from the JCRB Cell Bank, cell number: JCRB0134) was maintained in 5 mass% CO₂ at 37°C in growth medium (MEM from GIBCO) supplemented with 10 mass% fetal bovine serum, 1 mM of sodium pyruvate, 10 µg/ml of insulin, MEM non-essential amino acids (NEAA), 100 units/ml of penicillin, and 100 µg of streptomycin; and human pancreatic carcinoma-derived cell line (Capan-1 cell line, purchased from the ATCC Cell Bank, cell number: HTB-79) was maintained in 5 mass% CO₂ at 37°C in growth medium (IMDM from GIBCO) supplemented with 20 mass% fetal bovine serum, 100 units/ml of penicillin, and 100 µg of streptomycin.

### Example 9

### [Cell proliferation-suppressing activity tests on human BCL2 gene-targeted double-stranded nucleic acids]

The following experiments were performed to investigate *in vitro* cell proliferation-suppressing activities of human BCL2 gene-targeted double-stranded nucleic acids. Initially, the double-stranded nucleic acids BRDB-1 to 28 (28 in total) were prepared. Next, the human cancer-derived cell lines described in Examples 2 and 8 above were transfected with 10 nM each of these double-stranded nucleic acids using Lipofectamine 2000 (Invitrogen). The transfected cell lines were cultured for 72 hours after the transfection, and then the cell proliferation-suppressing activities of the double-stranded nucleic acids were measured in accordance with the cell proliferation assay method described in Example 3 above.

In addition, the cell proliferation assay was similarly performed with a double-stranded nucleic acid targeted to a non-target gene (GL3), instead of the above-described double-stranded nucleic acids.

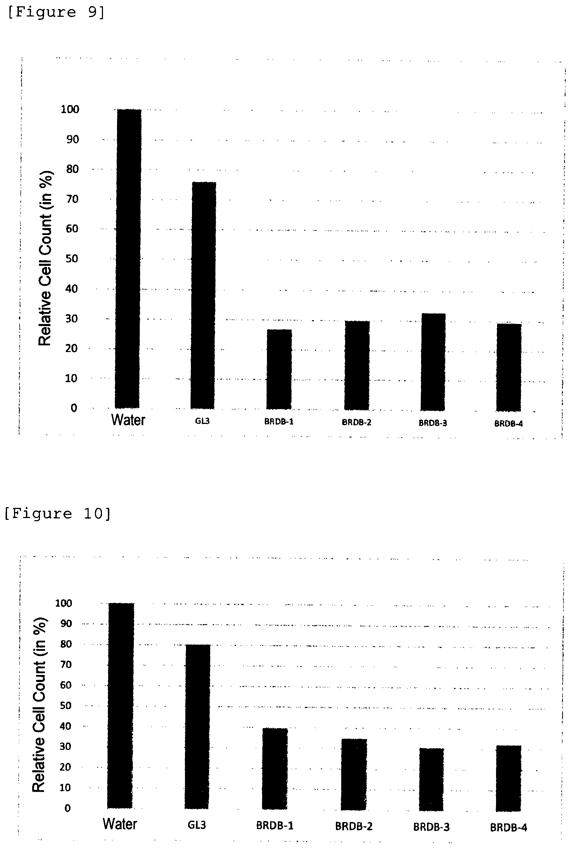

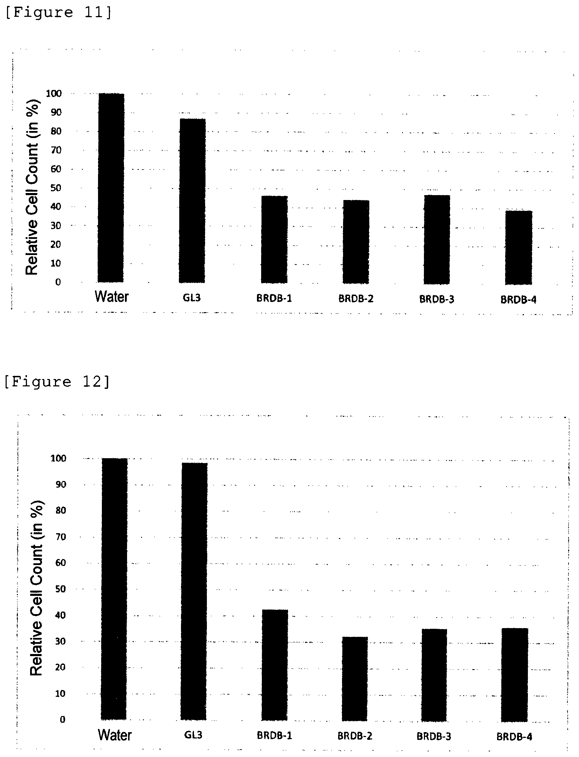

With regard to the proliferation-suppressing activities of the double-stranded nucleic acids BRDB-1 to 4 against the various cell lines, the results for PANC-1 cell line are shown in Figure 9, the results for Capan-1 cell line are shown in Figure 10, the results for Ls174T cell line are shown in Figure 11, and the results for HPAC cell line are shown in Figure 12.

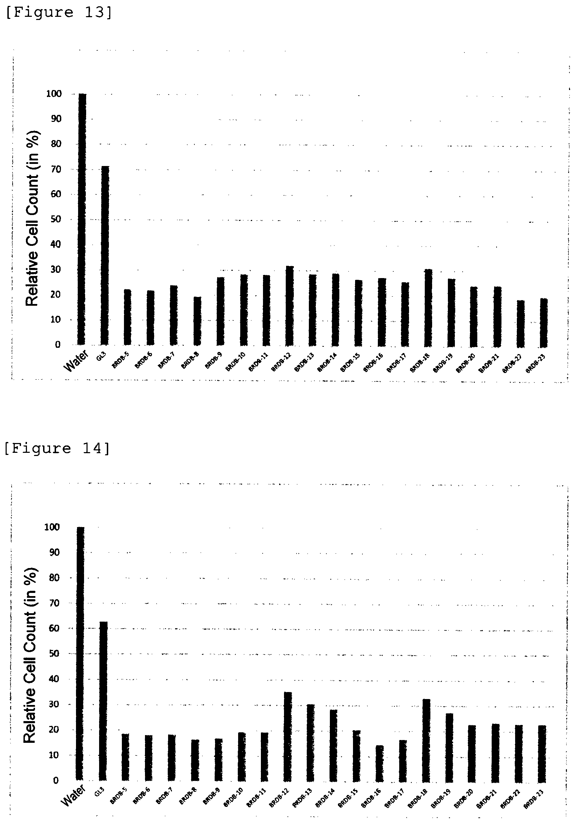

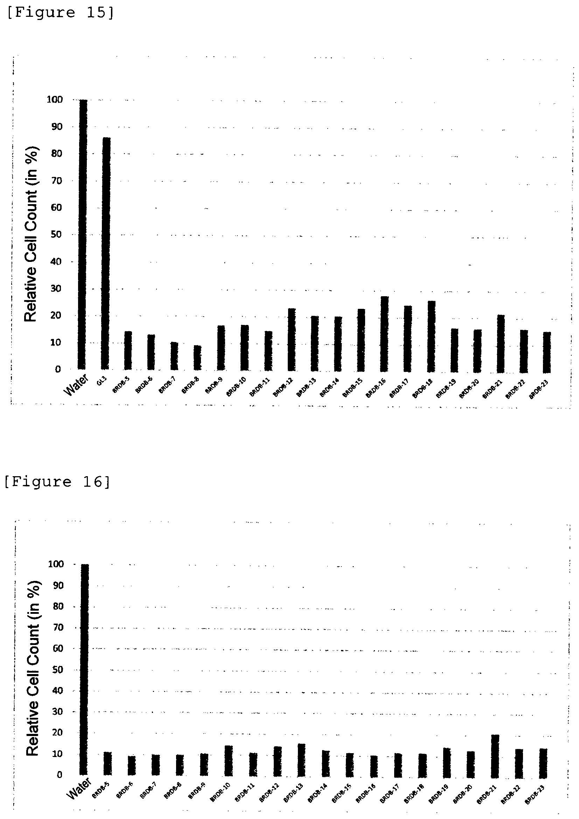

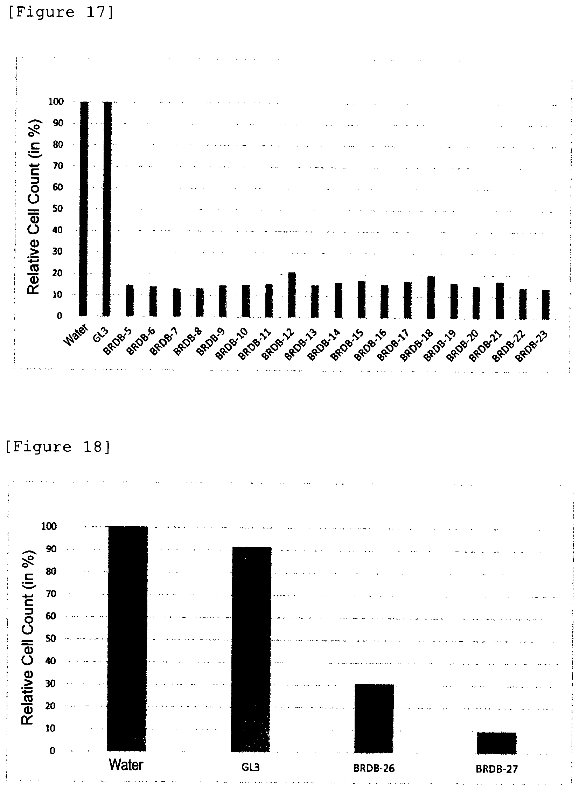

With regard to the proliferation-suppressing activities of the double-stranded nucleic acids BRDB-5 to 23 against the various cell lines, the results for AsPC-1 cell line are shown in Figure 13, the results for HCT116 cell line are shown in Figure 14, the results for A549 cell line are shown in Figure 15, the results for A431 cell line are shown in Figure 16, and the results for PANC-1 cell line are shown in Figure 17.

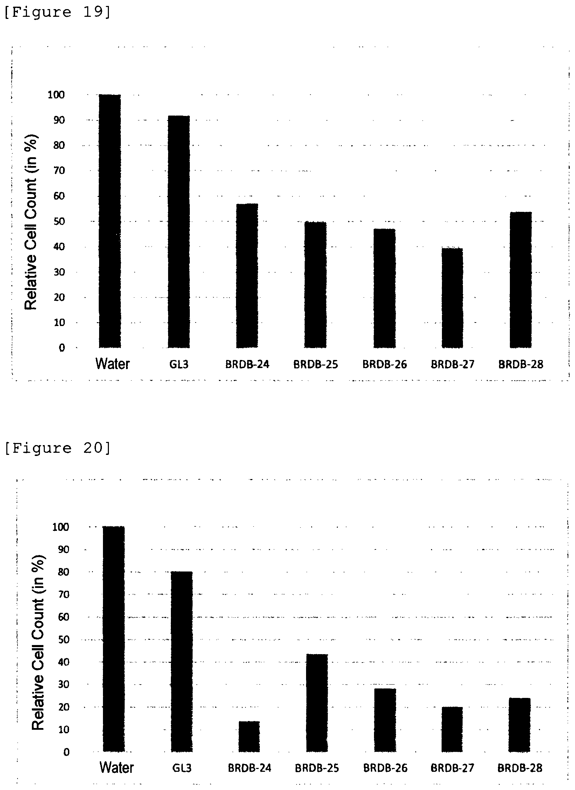

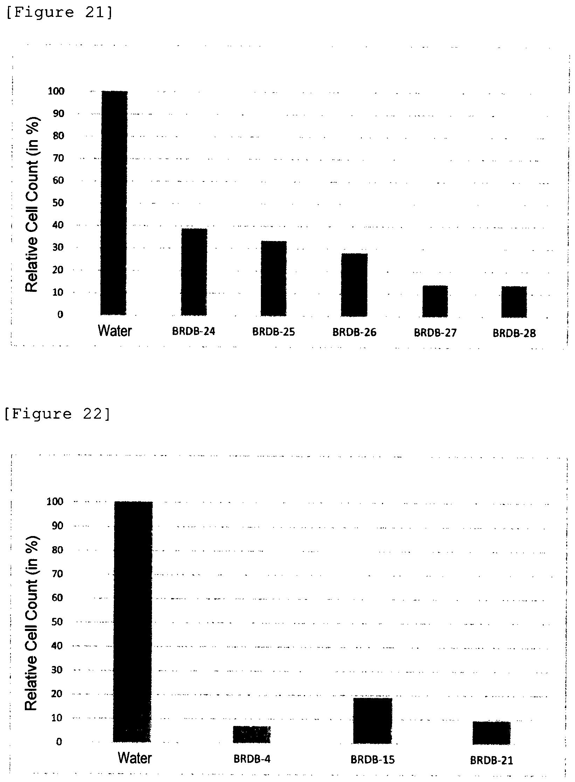

With regard to the proliferation-suppressing activities of the double-stranded nucleic acids BRDB-24 to 28 against the various cell lines, the results for A549 cell line are shown in Figure 18, the results for A431 cell line are shown in Figure 19, the results for AsPC-1 cell line are shown in Figure 20, and the results for HCT116 cell line are shown in Figure 21.

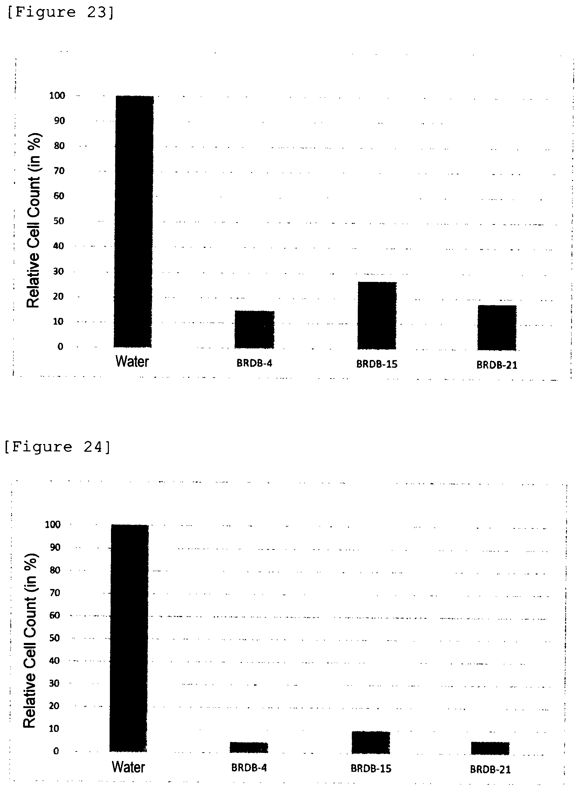

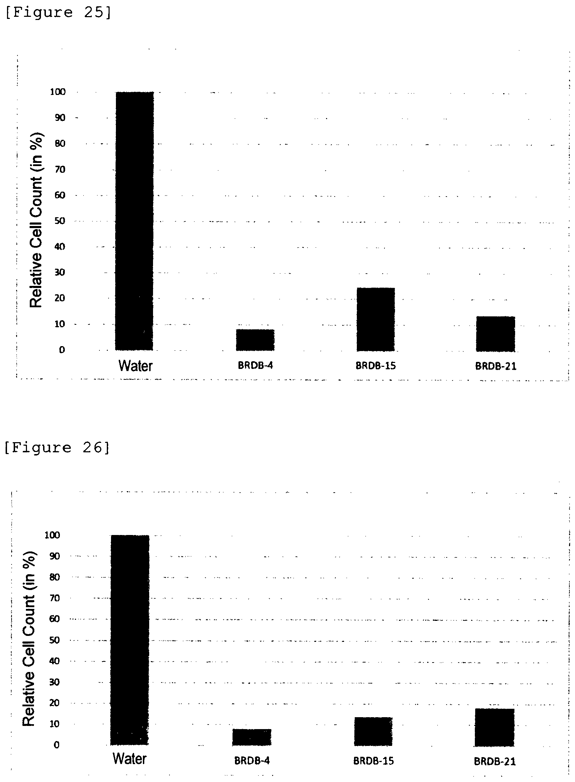

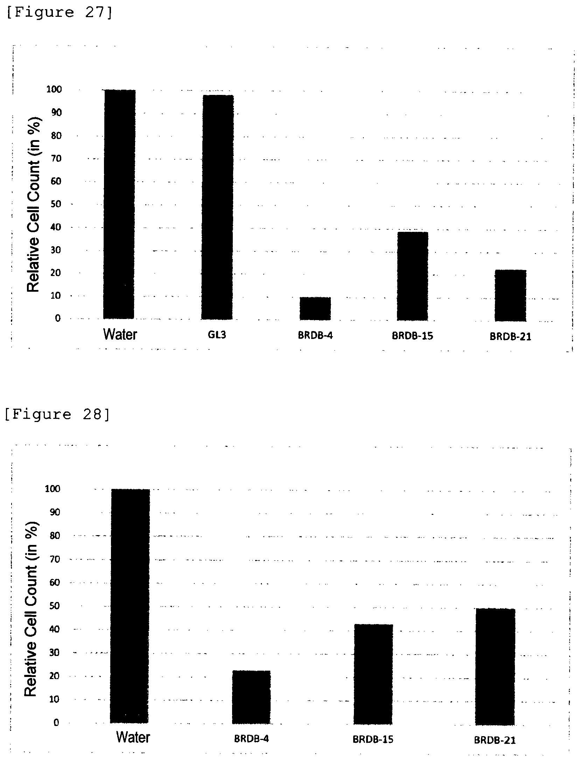

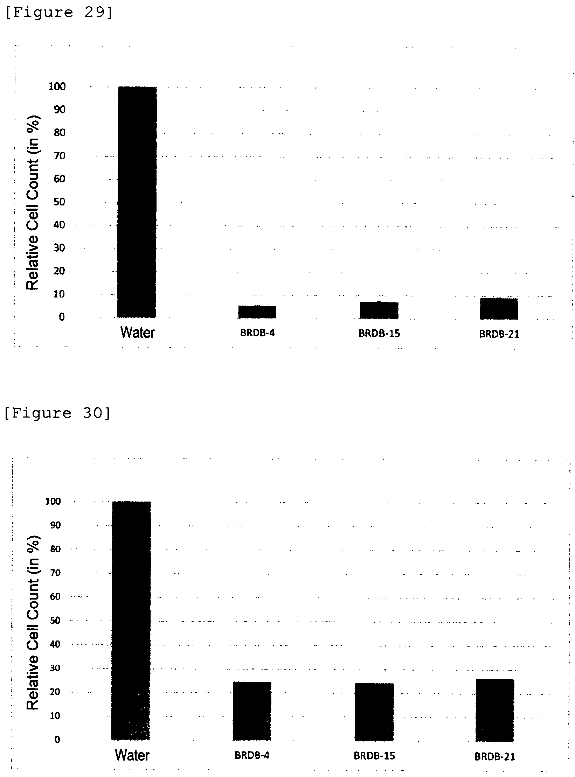

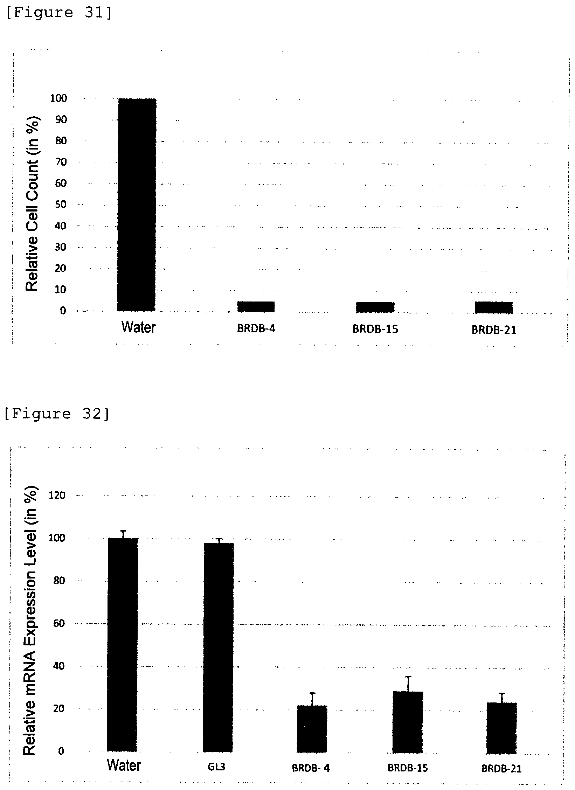

With regard to the proliferation-suppressing activities of the double-stranded nucleic acids BRDB-4, 15, and 21 against the various cell lines, the results for Caki-1 cell line are shown in Figure 22, the results for MCF-7 cell line are shown in Figure 23, the results for DU145 cell line are shown in Figure 24, the results for LNCaP cell line are shown in Figure 25, the results for PC-3 cell line are shown in Figure 26, the results for HGC27 cell line are shown in Figure 27, the results for MKN-45 cell line are shown in Figure 28, the results for OVCAR-3 cell line are shown in Figure 29, the results for HepG2 cell line are shown in Figure 30, and the results for T24 cell line are shown in Figure 31.

As seen from Figures 9 to 31, the double-stranded nucleic acids with various structures having a basic backbone of DNA-bulge-type DNA targeted to human BCL2 gene exhibited proliferation-suppressing activities against the various types of cancer cell lines.

### Example 10

### [mRNA expression-suppressing activity tests on human BCL2 gene-targeted double-stranded nucleic acids]

The following experiments were performed to investigate *in vitro* mRNA expression-suppressing activities of human BCL2 gene-targeted double-stranded nucleic acids. Initially, the double-stranded nucleic acids BRDB-4, 15, and 21 were prepared. Next, A549 cell lines described in Example 2 above were transfected with 10 nM each of these double-stranded nucleic acids using Lipofectamine 2000 (Invitrogen). The transfected cell lines were cultured for 24 hours after the transfection, and then the mRNA expression-suppressing activities of the double-stranded nucleic acids were quantified in accordance with the quantitative RT-PCR method described in Example 4 above.

In addition, the quantification of mRNA expression-suppressing activity was similarly performed with a double-stranded nucleic acid targeted to a non-target gene (GL3), instead of the above-described double-stranded nucleic acids.

The results of the mRNA expression-suppressing activities of the double-stranded nucleic acids BRDB-4, 15, and 21 against A549 cell line are shown in Figure 32.

As seen from Figure 32, the double-stranded nucleic acids having a basic backbone of DNA-bulge-type DNA targeted to human BCL2 gene exhibited mRNA expression-suppressing activities against A549 cell line.

### Example 11

### [Evaluation of the efficacy of a double-stranded nucleic acid having a basic backbone of DNA-bulge in pancreatic carcinoma cell line-derived liver metastasis mouse models]

The following experiments were performed to verify antitumor effects of a BCL2 gene-targeted double-stranded nucleic acid having a basic backbone of a DNA-bulge structure in pancreatic carcinoma cell line-derived liver metastasis mouse models.

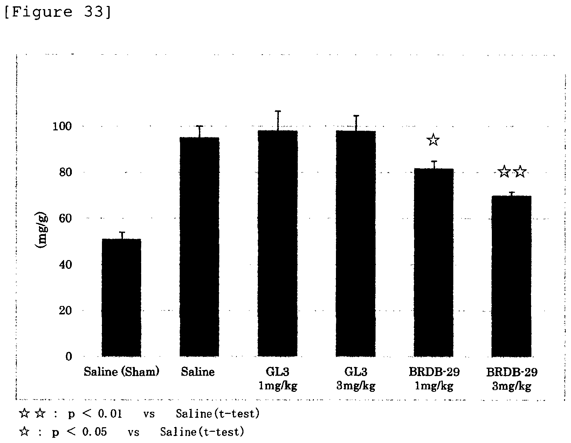

BALB/cA Jcl-nu/nu mice (six-week-old, male, purchased from CLEA Japan, Inc.) after several days of acclimation under rearing conditions were placed under general anesthesia by intraperitoneal administration of a mixture of three anesthetic agents (0.3 mg/kg of Domitor, 4 mg/kg of Dormicum, and 5 mg/kg of Vetorphale) at 80 µl per 10 grams of mouse body weight, and then injected with 1 x 10⁶ cells per mouse of human pancreatic carcinoma cell line (AsPC-1) in the spleen to produce intrasplenic injection liver metastasis mouse models (mouse models injected with PBS were also produced as shams). From the day following the implantation, body weight was measured every day (in the afternoon), and mice in a serious condition that experienced a sharp decrease in body weight (by 20% or more in several days) and were unable to walk on their own as well as unable to ingest food and water were euthanized in consideration of humane endpoints. On day 4 after the implantation of cancer cells, the mice were divided into groups based on body weight, and then saline, the double-stranded nucleic acid targeted to a non-target gene (GL3), or BRDB-29 was administered at a dose of 1 or 3 mg/kg to each mouse via the tail vein. The administration was carried out once a day (on days 4, 6, 8, 12, 15, 18, 20, 22, and 25), i.e., a total of nine times. Each mouse was followed up by measuring the body weight, for example, and, at 26 days after the implantation of cancer cells, each mouse was euthanized by cervical dislocation, and then the liver was removed. To evaluate the antitumor effect, the liver weight was measured and then corrected based on the mouse body weight to calculate the liver weight ratio. The results are shown in Figure 33. As seen from Figure 33, the BCL2 gene-targeted double-stranded nucleic acids having a basic backbone of DNA-bulge exhibited statistically significant antitumor effects in the pancreatic carcinoma cell line-derived liver metastasis mouse models.

### Example 12

### [Verification of MALAT1 gene knockdown effects in mouse livers of a double-stranded nucleic acid having a basic backbone of DNA-bulge]

*In vivo* experiments were performed to verify the utility of a double-stranded nucleic acid reagent according to one embodiment targeted to the non-coding RNA metastasis-associated lung adenocarcinoma transcript 1, MALAT1. As a control (ASO), a 16-mer single-stranded LNA/DNA gapmer (SEQ ID NO: 21) was used. This ASO contained three LNA nucleosides at the 5' end and three LNA nucleosides at the 3' end, as well as ten DNA nucleosides between them. This ASO gapmer was complementary to positions 1316 to 1331 of the non-coding RNA mouse metastasis-associated lung adenocarcinoma transcript 1 (MALAT1) (GenBank Accession number NR_002847).

Seven-week-old female C57BL/6 mice weighing from 20 to 25 g were used. PBS, the single-stranded antisense nucleic acid (ASO; SEQ ID NO: 21), or RBDB-30 was intravenously administered at a dose of 69.2 nmol/kg to each mouse via the tail vein (n = 5). Each mouse was perfused with PBS 72 hours after the administration, and then the mouse was dissected to remove the liver. Total RNA was extracted from the liver, and the mRNA expression-suppressing activity of the double-stranded nucleic acid was quantified in accordance with the quantitative RT-PCR method described in Example 4 above.

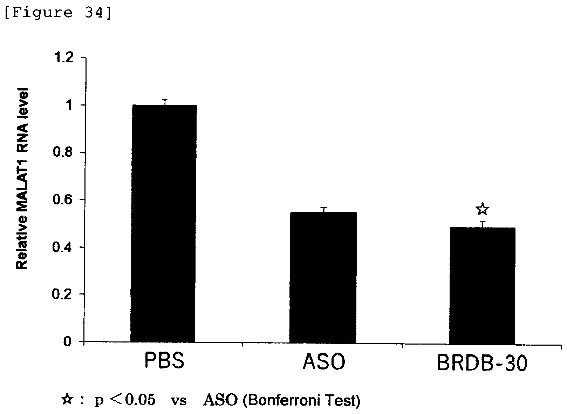

The results are shown in the graph of Figure 34. Both the nucleic acid reagents, i.e., the single-stranded ASO and RBDB-30, exhibited the inhibition of the expression of MALAT1 RNA, compared to the negative control (PBS only). However, the degree of inhibition achieved by RBDB-30 was higher than that achieved by the single-stranded ASO, and the difference was statistically significant.

### [Industrial Applicability]

As described above, through the use of the nucleic acid complex with at least one bulge structure of the present invention, the antisense nucleic acid can be delivered to a specific organ (cell) with high specificity and efficiency, and the nucleic acid can suppress the expression of a target gene or the level of a target transcript very effectively. Moreover, as a functional molecule to allow delivery to a specific organ, any of a variety of molecules such as lipids (for example, tocopherol and cholesterol), sugars (for example, glucose and sucrose), proteins, peptides, and antibodies can be applied to the nucleic acid complex, and thus, the nucleic acid complex can be targeted to any of various organs, tissues, and cells. Furthermore, even when the double-stranded nucleic acid of the nucleic acid complex of the present invention is modified to provide resistance to an RNase or the like, the antisense effect of the nucleic acid complex is not reduced, and thus, the nucleic acid complex is also applicable to enteral administration.

Furthermore, in the present invention, it has been found that, by introducing at least one bulge structure into a double-stranded nucleic acid complex comprising a DNA-based nucleic acid and a nucleic acid complementary to the nucleic acid, the double-stranded nucleic acid complex can achieve a superior antisense effect, and, for example, exhibits a target RNA expression inhibitory effect much superior to that achieved by an ASO having a single-stranded structure in an animal tissue, as demonstrated in the Examples.

The nucleic acid complex with at least one bulge structure of the present invention, therefore, is advantageous in that it can achieve high efficacy even when administered at a low concentration, and can exhibit reduced side effects by suppressing the distribution of the antisense nucleic acid in organs other than the target one, and hence, is useful as a pharmaceutical composition or the like for treating or preventing diseases associated with increased expression of a target gene and/or an increased level of a transcript, such as metabolic diseases, tumors, and infections.

## Claims

1. A nucleic acid complex comprising:
(i) an active moiety comprising a polynucleotide comprising at least one or more deoxyribonucleotides and optionally one or more modified nucleotides and/or nucleotide analogs as structural units; and
(ii) a carrier moiety comprising a polynucleotide comprising at least one or more nucleotides and optionally one or more modified nucleotides and/or nucleotide analogs as structural units, the polynucleotide being at least partially complementary to the polynucleotide of (i) the active moiety, wherein
the polynucleotide of (ii) the carrier moiety comprises at least one bulge.

2. The nucleic acid complex according to claim 1, which is a double-stranded nucleic acid complex.

3. The nucleic acid complex according to claim 1 or 2, wherein the polynucleotide of (ii) the carrier moiety comprises one to three bulges.

4. The nucleic acid complex according to claim 3, wherein the polynucleotide of (ii) the carrier moiety comprises one bulge.

5. The nucleic acid complex according to any one of claims 1 to 4, wherein a portion or all of the deoxyribonucleotides of the polynucleotide of (i) the active moiety are modified.

6. The nucleic acid complex according to any one of claims 1 to 5, wherein the polynucleotide of (i) the active moiety comprises one or more modified nucleotides and/or nucleotide analogs.

7. The nucleic acid complex according to any one of claims 1 to 6, wherein the polynucleotide of (i) the active moiety comprises one or more nucleotide analogs, and at least one of the nucleotide analogs is optionally modified.

8. The nucleic acid complex according to any one of claims 1 to 7, wherein the polynucleotide of (i) the active moiety comprises at least four contiguous deoxyribonucleotides, and
the polynucleotide comprises:
(a) a 5'-wing region that is positioned 5' to the at least four contiguous deoxyribonucleotides, and comprises one or more modified nucleotides and/or nucleotide analogs; and/or
(b) a 3'-wing region that is positioned 3' to the at least four contiguous deoxyribonucleotides, and comprises one or more modified nucleotides and/or nucleotide analogs.

9. The nucleic acid complex according to claim 8, wherein the polynucleotide of (i) the active moiety comprises (a) the 5'-wing region and (b) the 3'-wing region.

10. The nucleic acid complex according to claim 8 or 9, wherein the 5'-wing region comprises two to five modified nucleotides and/or nucleotide analogs, and the 3'-wing region comprises two to five modified nucleotides and/or nucleotide analogs.

11. The nucleic acid complex according to claim 8 or 9, wherein the 5'-wing region comprises one modified nucleotide and/or nucleotide analog, and the 3'-wing region comprises one modified nucleotide and/or nucleotide analog.

12. The nucleic acid complex according to any one of claims 8 to 11, wherein each of the 5'-wing region and the 3'-wing region comprises a sugar-modified nucleotide and/or a bridged nucleotide as a nucleotide analog.

13. The nucleic acid complex according to claim 12, wherein the bridged nucleotide is selected from the group consisting of LNA, cEt-BNA, amide BNA (AmNA), and cMOE-BNA.

14. The nucleic acid complex according to claim 13, wherein the bridged nucleotide is phosphorothioated.

15. The nucleic acid complex according to any one of claims 8 to 14, wherein the at least four contiguous deoxyribonucleotides are phosphorothioated.

16. The nucleic acid complex according to any one of claims 8 to 15, wherein the polynucleotide of (i) the active moiety consists of a 5'-wing region consisting of one or more phosphorothioated bridged nucleotides, phosphorothioated deoxyribonucleotides, and a 3'-wing region consisting of one or more phosphorothioated bridged nucleotides.

17. The nucleic acid complex according to claim 8 or 9, wherein the 5'-wing region consists of one to five sugar-modified ribonucleotides, and the 3'-wing region consists of one to five sugar-modified ribonucleotides, and wherein the sugar-modified ribonucleotides are optionally phosphorothioated.

18. The nucleic acid complex according to claim 17, wherein the polynucleotide of (i) the active moiety consists of a 5'-wing region consisting of one or more phosphorothioated sugar-modified ribonucleotides, at least four contiguous phosphorothioated deoxyribonucleotides, and a 3'-wing region consisting of one or more phosphorothioated sugar-modified ribonucleotides.

19. The nucleic acid complex according to claim 17 or 18, wherein the sugar modification is selected from the group consisting of 2'-O-methylation, 2'-O-methoxyethylation (2'-MOE modification), 2'-O-aminopropylation (2'-AP modification), and 2'-fluorination.

20. The nucleic acid complex according to any one of claims 1 to 5, wherein the polynucleotide of (i) the active moiety consists of at least four contiguous phosphorothioated deoxyribonucleotides.

21. The nucleic acid complex according to any one of claims 1 to 20, wherein the polynucleotide of (ii) the carrier moiety comprises one or more deoxyribonucleotides as structural units.

22. The nucleic acid complex according to claim 21, wherein the polynucleotide of (ii) the carrier moiety consists of one or more deoxyribonucleotides.

23. The nucleic acid complex according to any one of claims 1 to 21, wherein the polynucleotide of (ii) the carrier moiety comprises one or more ribonucleotides as structural units.

24. The nucleic acid complex according to claim 21 or 23, wherein the polynucleotide of (ii) the carrier moiety consists of one or more ribonucleotides and one or more deoxyribonucleotides.

25. The nucleic acid complex according to claim 23 or 24, wherein the ribonucleotides are unmodified.

26. The nucleic acid complex according to any one of claims 21 to 25, wherein the deoxyribonucleotides are unmodified.

27. The nucleic acid complex according to any one of claims 21 to 25, wherein a portion or all of the deoxyribonucleotides are modified.

28. The nucleic acid complex according to claim 27, wherein the modified deoxyribonucleotides are base-modified deoxyribonucleotides.

29. The nucleic acid complex according to claim 28, wherein the base-modified deoxyribonucleotides are abasic deoxyribonucleotides.

30. The nucleic acid complex according to any one of claims 27 to 29, wherein the modified deoxyribonucleotides have no modifications other than the base modification.

31. The nucleic acid complex according to any one of claims 1 to 30, wherein the bulge is formed of one or more deoxyribonucleotides and/or ribonucleotides.

32. The nucleic acid complex according to claim 31, wherein the bulge is formed of one or more deoxyribonucleotides.

33. The nucleic acid complex according to claim 32, wherein the bulge is formed of one or more unmodified deoxyribonucleotides.

34. The nucleic acid complex according to claim 32, wherein the bulge is formed of one or more base-modified deoxyribonucleotides.

35. The nucleic acid complex according to claim 31, wherein the bulge is formed of one or more ribonucleotides.

36. The nucleic acid complex according to claim 35, wherein the bulge is formed of one or more unmodified ribonucleotides.

37. The nucleic acid complex according to any one of claims 1 to 36, wherein the polynucleotide of (i) the active moiety comprises at least one mismatch compared to the polynucleotide of (ii) the carrier moiety and/or a target transcript.

38. The nucleic acid complex according to any one of claims 1 to 36, wherein the polynucleotide of (i) the active moiety comprises no mismatch compared to the polynucleotide of (ii) the carrier moiety and/or a target transcript.

39. The nucleic acid complex according to any one of claims 1 to 38, wherein the polynucleotide of (ii) the carrier moiety comprises at least one mismatch compared to the polynucleotide of (i) the active moiety.

40. The nucleic acid complex according to any one of claims 1 to 38, wherein the polynucleotide of (ii) the carrier moiety comprises no mismatch compared to the polynucleotide of (i) the active moiety.

41. The nucleic acid complex according to any one of claims 1 to 40, wherein the polynucleotide of (i) the active moiety is 8 to 100 bases in length.

42. The nucleic acid complex according to any one of claims 1 to 41, wherein the polynucleotide of (ii) the carrier moiety is 8 to 100 bases in length.

43. The nucleic acid complex according to any one of claims 1 to 42, wherein the polynucleotide of (i) the active moiety is identical in length to the polynucleotide of (ii) the carrier moiety when compared with the bulge being excluded from the polynucleotide of (ii) the carrier moiety.

44. The nucleic acid complex according to any one of claims 1 to 42, wherein the polynucleotide of (i) the active moiety is different in length from the polynucleotide of (ii) the carrier moiety when compared with the bulge being excluded from the polynucleotide of (ii) the carrier moiety.

45. The nucleic acid complex according to any one of claims 1 to 44, wherein (ii) the carrier moiety further comprises a functional moiety having a function selected from a labeling function, a purification function, and a targeted delivery function.

46. The nucleic acid complex according to claim 45, wherein (ii) the carrier moiety comprises the functional moiety having the targeted delivery function, and the functional moiety is a molecule selected from the group consisting of lipids, peptides, proteins, sugar chains, small molecules, and biomolecules/bioactive molecules.

47. The nucleic acid complex according to claim 46, wherein the functional moiety is a peptide containing a cyclic arginine-glycine-aspartic acid (RGD) sequence, N-acetylgalactosamine, cholesterol, vitamin E (tocopherol), stearic acid, docosanoic acid, anisamide, folic acid, anandamide, or spermine.

48. The nucleic acid complex according to any one of claims 1 to 44, wherein (ii) the carrier moiety does not comprise a functional moiety having a targeted delivery function.

49. A double-stranded nucleic acid complex comprising:
(i) an active moiety comprising a polynucleotide comprising, as structural units, at least four contiguous phosphorothioated deoxyribonucleotides, a 5'-wing region that is positioned 5' to the at least four contiguous deoxyribonucleotides, and consists of two to five phosphorothioated bridged nucleotides, and a 3'-wing region that is positioned 3' to the at least four contiguous deoxyribonucleotides, and consists of two to five phosphorothioated bridged nucleotides; and
(ii) a carrier moiety comprising a polynucleotide consisting of one or more unmodified ribonucleotides and one or more unmodified deoxyribonucleotides as structural units, the polynucleotide being at least partially complementary to the polynucleotide of (i) the active moiety, wherein
the polynucleotide of (ii) the carrier moiety comprises at least one bulge, and the bulge is formed of one or more unmodified ribonucleotides,
the polynucleotide of (i) the active moiety comprises no mismatch compared to the polynucleotide of (ii) the carrier moiety and/or a target transcript, and
(ii) the carrier moiety does not comprise a functional moiety having a targeted delivery function.

50. The nucleic acid complex according to any one of claims 1 to 49, for use in reducing expression of a target gene in a mammal.

51. The nucleic acid complex according to claim 50, wherein the polynucleotide of the active moiety is an antisense strand complementary to any region of mRNA of the target gene.

52. The nucleic acid complex according to any one of claims 1 to 49, for use in reducing expression of a non-protein-coding transcript in a mammal.

53. The nucleic acid complex according to claim 52, wherein the polynucleotide of the active moiety is an antisense strand complementary to any region of the transcript.

54. A pharmaceutical composition comprising the nucleic acid complex according to any one of claims 1 to 49 and optionally a pharmacologically acceptable carrier.

55. A method for reducing the level of a transcript in a cell comprising the step of contacting the nucleic acid complex according to any one of claims 1 to 49 with the cell, wherein
the polynucleotide of the active moiety is an antisense strand complementary to any region of the transcript.

56. The method according to claim 55, wherein the transcript is a protein-coding mRNA transcript.

57. The method according to claim 55, wherein the transcript is a non-protein-coding transcript.

58. Use of the nucleic acid complex according to any one of claims 1 to 49 for reducing expression of a target gene in a mammal.

59. Use of the nucleic acid complex according to any one of claims 1 to 49 for the manufacture of a medicament for reducing expression of a target gene in a mammal.

60. A method for reducing the expression level of a target gene in a mammal comprising the step of administering the nucleic acid complex according to any one of claims 1 to 49 to a mammal, wherein
the polynucleotide of the active moiety is an antisense strand complementary to any region of mRNA of the target gene.

61. The method according to claim 60, wherein the administration is enteral administration.

62. The method according to claim 60, wherein the administration is non-enteral administration.

63. The method according to any one of claims 60 to 62, wherein the nucleic acid complex is administered at a dose of 0.001 to 50 mg/kg/day.

64. The method according to any one of claims 60 to 63, wherein the mammal is a human.
